# EUROPEAN PATENT APPLICATION

(11) **EP 3 892 737 A1**
(43) Date of publication of application: **13.10.2021**
(21) Application number: 20169080.7
(22) Date of filing: 09.04.2020
(51) Int. Cl.: C12Q 1/6869, C12Q 1/70

(54) **QUALITATIVE AND QUANTITATIVE DETERMINATION OF SINGLE VIRUS HAPLOTYPES IN COMPLEX SAMPLES**

(71) Applicant: Takeda Vaccines, Inc., Cambridge, MA 02139 (US)
(72) Inventor: DEAN, Hansi, Cambridge, MA 02139 (US); KARWAL, Lovkesh, Cambridge, MA 02139 (US); LIVENGOOD, Jill A., Cambridge, MA 02139 (US); DAS, Subash, Cambridge, MA 02139 (US)
(74) Representative: Lasar, Andrea Gisela

(57) **Abstract**

The present invention provides methods for detecting the presence of at least one revertant virus in a sample containing an attenuated virus by single-molecule real-time (SMRT) sequencing. It further provides methods for the quantitative analysis of a mixture of virus haplotypes in a virus sample by SMRT sequencing. It also provides kits useful in performing said methods.

## Description

### Technical field

The present invention relates to methods for detecting the presence of at least one revertant virus in a sample containing an attenuated virus. It also relates to methods for the quantitative analysis of a mixture of virus haplotypes in a virus sample. It further relates to kits useful in performing said methods.

### Background of the Invention

Dengue virus is an arbovirus in the *Flaviviridae* family. It is an enveloped virus with an isometric capsid and a linear positive sense RNA genome. Dengue viruses are endemic in most regions of the tropics and are transmitted by infectious mosquitoes, typically *Aedes aegypti,* causing symptomatic infections or asymptomatic seroconversion. Symptomatic dengue infection is a systemic and dynamic disease. It has a wide clinical spectrum that includes both severe and non-severe clinical manifestations. There are four different dengue serotypes (DENV 1-4) which are genetically related to yellow fever and similar tick-borne encephalitis viruses.

One tetravalent dengue vaccine (TDV) presently tested in clinical trials consists of all four dengue serotypes, TDV1, TDV2, TDV3 and TDV4. TDV2 is a live attenuated recombinant virus that originated from PDK-53 that became non-pathogenic after continuous passaging of DEN-2 virus in primary dog kidney (PDK) cells. The mutations necessary and sufficient for the attenuated phenotype of TDV2 virus have been genetically identified (Butrapet et al., J. Virol., vol. 74, no. 7 (2000), 3011-3019). These mutations are C-to-T at genomic nucleotide position 57 in the 5' noncoding region, and Gly-to-Asp and Glu-to- Val mutations at amino acid positions 53 and 250 in nonstructural proteins (NS) 1 and 3, respectively. This corresponds to a C to T mutation at genomic nucleotide position 57, a G- to A mutation at genomic nucleotide position 2579 and an A to T mutation at genomic nucleotide position 5270. These three genetic determinants of attenuation reside outside of the structural gene regions of the TDV2 viral genome. TDV1, TDV3 and TDV4 are chimeric variants having the backbone of Dengue virus 2 PDK53 and the prM and E proteins of Dengue virus 1, Dengue virus 3 and Dengue virus 4, respectively, which maintain the attenuation phenotype of TDV-2.

TDV is comprised of four viruses when administered into humans. Host immune response is triggered due to vaccine virus replication. Amplification of virus may generate mutations leading to reversion to wild type. In a scenario when there are febrile cases in the first 30 days post vaccination, it is important to confirm that these illnesses are not caused by vaccine viruses that may have been reverted to wild type and became pathogenic, but may be due to an infection by other closely related Flaviviruses or any other microbial agents. In such a complicated scenario it is important to decipher and identify the genome variants present in the suspected sample. This requires sequencing methods that can accurately capture the genetic diversity of RNA virus genomes.

Huang et al., PLOS Negl. Trop. Diseases 7 (2013), e2243, 1-11 relates to the genetic and phenotypic characterization of manufacturing seeds of a tetravalent Dengue vaccine (DENVax). For sequencing of the virus Sanger sequencing on an automated sequencer was used. For testing of the reversion at position 5'NC-57 locus of the vaccine a Taqman-based mismatch amplification mutation assay (Taq-MAMA) was used. This represents an improved quantitative SNP assay. According to the authors the detection sensitivity could be lowered to detect 0.01-0.07% reversion. In addition, possible revertants have been tested in a murine neurovirulence model.

The prior art therefore suffers from the drawbacks that no sequence information of entire single virus haplotypes such as revertants can be provided. Due to the lack of identification of entire single virus haplotypes the prior art cannot quantify the amount of entire single virus haplotypes in complex mixtures such as patient samples or live attenuated vaccines.

In view thereof, there is a need for the provision of improved methods for the detection of revertants in complex samples containing live attenuated viruses, in particular Dengue virus. Further, there is a need for the quantitation of single viral haplotypes in complex samples.

### Summary of the Invention

The technical problems underlying the invention are solved by the provision of the subject-matter as defined in the claims.

According to a first aspect, a method for detecting the presence of at least one revertant virus in a sample containing an attenuated virus comprising the steps:
a) purifying virus nucleic acids from said sample;
b) optionally, preparing double-stranded DNA templates from said virus nucleic acids;
c) amplifying said viral nucleic acids of a) or said double-stranded DNA templates of b) by polymerase chain reaction (PCR) such that PCR products having a length of at least 2 kb and comprising at least one attenuation locus of said attenuated virus are generated;
d) sequencing said PCR products by single-molecule real-time (SMRT) sequencing; and
e) comparing at least one nucleotide at said at least one attenuation locus in said PCR products with the corresponding nucleotide in the wild-type virus, wherein the presence of the wild-type nucleotide at said at least one attenuation locus is indicative of the presence of a revertant virus in said sample.

The inventors have found that SMRT sequencing (PacBio sequencing) is useful for the qualitative and quantitative determination of revertants within a sample of live attenuated virus vaccine. SMRT sequencing was known to exhibit a high sequencing error rate of 13 % e.g. in comparison to a low sequencing error rate of 0.001 % of the conventional Sanger method. Due to the use of high number or Read counts and additionally high number of UMI counts the PacBio inherent high sequence error is significantly lowered. The successful use of SMRT sequencing for the determination of single or multiple revertants in a sequence having a length of more than 5 kb, wherein the revertants occur in low quantities in a live attenuated vaccine sample may be considered as a surprising advantageous effect. The use of SMRT sequencing provides the advantage of a high read length which is useful for covering the entire Dengue virus genome. A further advantage of SMRT sequencing of live attenuated vaccine samples over known approaches such as three separate PCR sequencing reactions is that the entire sequence of a single haplotype can be determined in comparison to an "average" haplotype when conventional PCR approaches are used to determine the sequence at the attenuation loci. An average haplotype means that the sequence is not determined by sequencing of a single nucleic acid molecule, but by sequencing of different nucleic acid molecules which cover different parts of the complete nucleic acid molecule. Hence, the nucleic acid sequence determined at the second or third attenuation locus may not be derived from the same nucleic acid molecule used for determining the sequence at the first attenuation locus. While Sanger sequencing is useful for determining the presence of a revertant in a sample comprising different nucleic acid molecules, it cannot determine the whole nucleic acid sequence of the revertant in one sequencing reaction.

SMRT sequencing was known to exhibit lower throughput compared to second generation sequencing technologies. This was considered an obstacle in quantitative analysis, such as gene/isoform abundance estimation. To overcome this obstacle third generation sequencing technologies such as SMRT may be evaluated as hybrid sequencing, i.e. a combination of second generation and third generation sequencing technology data. It was therefore surprising that the use of SMRT sequencing alone was sufficient to provide a quantitative analysis of complex mixtures of viruses, such as Dengue viruses.

According to a second aspect, a method for the quantitative analysis of a mixture of virus haplotypes in a virus sample is provided comprising the steps:
a) purifying virus nucleic acids from said sample;
b) optionally, preparing double-stranded DNA templates from said virus nucleic acids by using a primer comprising a unique molecular identifier (UMI) sequence such that said double-stranded DNA templates comprise the UMI sequence;
c) amplifying said double-stranded DNA templates by polymerase chain reaction (PCR) such that PCR products having a length of at least 2kb and comprising a unique molecular identifier (UMI) sequence are generated;
d) sequencing said PCR products by single-molecule real-time (SMRT) sequencing; and
e) determining the relative amounts of said PCR products and thereby the virus haplotypes contained in the sample.

Surprisingly, the inventors have found that a UMI workflow using the above method identified 8 to 11 haplotypes per revertant, i.e. is more sensitive compared to a BLASR or Quiver workflow which only identified 2 to 5 haplotypes per revertant on the basis of the same sample. Samples of the Dengue virus reference monorevertants for reversion at positions NS1-2579 (P1), NS3-5270 (P3) and 5'NC-57 (P5 and P51), respectively, were obtained from Center of Disease Control and Prevention (CDC). In said reference samples the UMI workflow identified significantly more individual haplotypes compared to the BLASR or the Quiver workflow.

According to a third aspect the use of the method according to the first aspect in the quality control of vaccines containing live, attenuated virus is provided.
According to a fourth aspect the use of the method according to the second aspect in the diagnosis of a virus infection in a patient sample is provided.

According to a fifth aspect is provided a kit for detecting the presence of at least one revertant virus in a sample containing an attenuated dengue virus comprising a first primer pair comprising the nucleotide sequence of SEQ ID NO: 9 (5F) and a second primer comprising the nucleotide sequence of SEQ ID NO: 6 (3R).

According to a sixth aspect is provided a kit for the quantitative analysis of a mixture of virus haplotypes in a dengue virus sample comprising a first primer comprising the nucleotide sequence of SEQ ID NO:21 (Primer IIA) and a second primer comprising the nucleotide sequence of SEQ ID NO:9 (5F).

### Brief Description of the Drawings

- Figure 1: is a chart showing the SMRT (Pacbio) sequencing work flow.
- Figure 2: shows the TDV2 genome.
- Figure 3: shows the positions of primers on TDV2 genome used for Sanger sequencing.
- Figure 4: shows the mapped reads of Insert Length.

### Detailed Description of the Invention

Where the term "comprise" or "comprising" is used in the present description and claims, it does not exclude other elements or steps. For the purpose of the present invention, the term "consisting of' is considered to be an optional embodiment of the term "comprising". If hereinafter a group is defined to comprise at least a certain number of embodiments, this is also to be understood to disclose a group which optionally consists only of these embodiments. Where an indefinite or a definite article is used when referring to a singular noun e.g. "a" or "an", "the", this includes a plural form of that noun unless specifically stated. *Vice versa,* when the plural form of a noun is used it refers also to the singular form.

Furthermore, the terms first, second, third or (a), (b), (c) and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In the context of the present invention any numerical value indicated is typically associated with an interval of accuracy that the person skilled in the art will understand to still ensure the technical effect of the feature in question. As used herein, the deviation from the indicated numerical value is in the range of ± 10%, and preferably of ± 5%. The aforementioned deviation from the indicated numerical interval of ± 10%, and preferably of ± 5% is also indicated by the terms "about" and "approximately" used herein with respect to a numerical value.

According to the first aspect of the present invention is provided a method for detecting the presence of at least one revertant virus in a sample containing an attenuated virus comprising the steps:
a) purifying virus nucleic acids from said sample;
b) optionally, preparing double-stranded DNA templates from said virus nucleic acids;
c) amplifying said viral nucleic acids of a) or said double-stranded DNA templates of b) by polymerase chain reaction (PCR) such that PCR products having a length of at least 2 kb and comprising at least one attenuation locus of said attenuated virus are generated;
d) sequencing said PCR products by single-molecule real-time (SMRT) sequencing; and
e) comparing at least one nucleotide at said at least one attenuation locus in said PCR products with the corresponding nucleotide in the wild-type virus sequence, wherein the presence of the wild-type nucleotide at said at least one attenuation locus is indicative of the presence of a revertant virus in said sample.

"Virus" herein means any virus including double-stranded and single-stranded DNA viruses, and double and single-stranded RNA viruses.

"An attenuated virus" herein means that the virus has a reduced replication capacity and/or a reduced infectivity in host cells compared to wild-type virus. The replication capacity and/or infectivity may be determined *in vitro* in suitable cell systems or *in vivo* in suitable animal models. Attenuation may be achieved by serial passaging of the virus in a foreign host such as in tissue culture, embryonated eggs or live animals. Alternatively, attenuation may be performed by chemical agents.

Live attenuated viruses are important for use in viral vaccines, since the live attenuated virus generates a stronger immune response compared to an inactivated virus. The live attenuated virus may be an RNA virus or a DNA virus. Suitable live attenuated RNA viruses may be selected from dengue virus, poliovirus, rubella virus, measles virus, yellow fever virus, mumps virus and influenza virus. Preferably, the live attenuated RNA virus is dengue virus. Suitable live attenuated DNA viruses include varicella zoster virus, vaccinia virus, smallpox virus, Herpes simplex virus and chikungunya virus. The live attenuated virus may differ from the wild-type virus in one or more mutations in the nucleic acid sequence of the virus. For example, for one tetravalent dengue vaccine (TDV) it is known that attenuation of neurovirulence in newborn mice is determined by mutations 5'NCR-57 C->U, NS1-53 Gly->Asp (nt-2579 G->A) and NS3-250 Glu->Val (nt-5270 A->U) (Butrapet et al., J. Virol. 74 (2000), 3011-3019). The above nucleotide sequence numbering relates to the nucleotide sequence of Dengue virus serotype 2 (SEQ ID NO:23).

"A revertant virus" herein means a virus derived from an attenuated virus having a reversion of a nucleotide at one or more attenuation loci to the wild-type sequence. The detection of revertant virus is of particular importance for the quality control of vaccines on the basis of live attenuated viruses. In the case of TDV, a revertant will have nucleotide C at position 57, nucleotide G at position 2579 and/or nucleotide A on position 5270.

"Sample" herein means any sample from vaccinated individuals or patients or a sample to be tested at the stage of quality control of manufactured vaccines. The sample may be from whole blood or serum, most preferred the samples are from serum. The sample may also be vaccine composition or the stock solution prior to administration. This may be important for quality control and safety reasons.
Preferably a vaccine composition to be analysed according to the present invention comprises a dengue antigen of each of serotypes 1 to 4 which are each independently selected from the group consisting of: (a) a live attenuated dengue virus and (b) a live attenuated chimeric virus.

Preferably a vaccine composition to be analysed according to the present invention comprises a dengue antigen of each of serotypes 1 to 4, wherein said dengue antigens of serotypes 1 , 3 and 4 are each a live attenuated chimeric dengue virus and said dengue antigen of serotype 2 is selected from the group consisting of a live attenuated dengue virus and a live attenuated chimeric dengue virus. For example, a vaccine composition to be analysed of the present invention may comprise a dengue antigen of each of serotypes 1 to 4, wherein said dengue antigens of serotypes 1 , 3 and 4 are each a live attenuated chimeric dengue/dengue virus and said dengue antigen of serotype 2 is a live attenuated dengue virus. For example, a vaccine composition to be analysed according to the present invention may be the tetravalent mixture of dengue antigens of each of serotypes 1 to 4 (referred to as DENVax) which is disclosed in Huang et al., PLoS Negl Trop Dis 7(5): e2243 (2013). Alternatively, a vaccine composition to be analysed according to the present invention may comprise a dengue antigen of each of serotypes 1 to 4, wherein said dengue antigens of serotypes 1 , 3 and 4 are each a live attenuated chimeric YF/dengue virus and said dengue antigen of serotype 2 is a live attenuated dengue virus.

Preferably a vaccine composition to be analysed according to the present invention comprises a dengue antigen of each of serotypes 1 to 4, wherein said dengue antigens of serotypes 1 , 3 and 4 are each independently selected from the group consisting of: (a) a live attenuated dengue virus and (b) a live attenuated chimeric dengue virus and said dengue antigen of serotype 2 is a live attenuated chimeric dengue virus. For example, a vaccine composition to be analysed according to the present invention may comprise a dengue antigen of each of serotypes 1 to 4, wherein said dengue antigens of serotypes 1 , 3 and 4 are each a live attenuated dengue virus and said dengue antigen of serotype 2 is a live attenuated chimeric dengue/dengue virus. For example, a vaccine composition according to the present invention may be any of the tetravalent mixtures of dengue antigens of each of serotypes 1 to 4 (referred to as TV001, TV002, TV003 and TV004') which are disclosed in Durbin et al., Journal of Infectious Diseases (2013), 207, 957-965. Preferably, a vaccine composition to be analysed according to this embodiment of the invention is TV003.

Preferably a vaccine composition to be analysed according to the present invention comprises a dengue antigen of each of serotypes 1 to 4, wherein each of said dengue antigens is a live attenuated chimeric dengue virus, preferably a chimeric YF/dengue virus, more preferably a chimeric YF/dengue virus which comprises an attenuated YF genomic backbone whose prM-E sequence has been substituted with the prM-E sequence of dengue virus.

Preferably, a live attenuated chimeric dengue virus to be analysed according to the present invention comprises one or more proteins from a dengue virus and one or more proteins from a different flavivirus. Preferably, the different flavivirus is an yellow fever virus (i.e. a chimeric YF/dengue virus). Preferably a live attenuated chimeric dengue virus to be analysed according to the present invention comprises an attenuated yellow fever virus genome whose prM-E sequence has been substituted with the prM-E sequence of a dengue virus. Alternatively, a live attenuated chimeric dengue virus to be analysed according to the present invention comprises one or more proteins from a first dengue virus and one or more proteins from a second dengue virus (i.e. a chimeric dengue/dengue virus). Preferably said first dengue virus and said second dengue virus are of different serotypes. Where said first dengue virus and said second dengue virus are of the same serotype, said first and second dengue viruses are different strains.

If necessary, the virus concentration in the sample may be increased by growing the virus in suitable host cells. Suitable host cells include mammalian cells such as e.g. Vero cells. It is preferred that Vero cells are seeded in T25 cm² flasks at 1.5e⁶ cells/flask and grown overnight to 80-90% confluency by incubating at 37°C with 5% CO₂ tension. Preferably, the virus is diluted in assay medium DMEM containing 1% FBS and 1% Penicillin/Streptomycin. Preferably, Vero cells are infected with TDV at 0.1MOI and allowed virus adsorption for 1 hour at 37°C 5% CO₂ incubator. For obtaining high quality viral RNA a preferred method is described in the Examples section.

"Purifying virus nucleic acids from the sample" may be performed using any method for DNA or RNA isolation known in the art. Any method known in the art may be used for DNA extraction or purification. Suitable methods comprise *inter alia* steps such as centrifugation steps, precipitation steps, chromatography steps, dialyzing steps, heating steps, cooling steps and/or denaturation steps.

"Preparing double-stranded DNA templates" herein means that if the virus nucleic acid is an RNA, the method further includes a step of reverse transcription with a reverse transcriptase, a reverse primer and a mixture of nucleotides such that a RNA/DNA hybrid is generated which is subsequently converted into a double-stranded cDNA template. The reaction may be carried out in an automated thermal cycler. Preferably, for RNA purified from Dengue virus the reverse transcription is performed using as reverse primer an oligonucleotide having the nucleotide sequence of SEQ ID NO:6 at a temperature in the range from about 45°C to about 65° C, preferably at a temperature in the range from about 50°C to about 55°C.

If the virus nucleic acid is a single-stranded DNA, the double-stranded DNA-template is generated by the activity of a template-dependent DNA polymerase. If the virus nucleic acid purified from the virus is already a double-stranded nucleic acid, there is no need for this optional step.

"Amplifying said viral nucleic acids of step a) or step b) such that PCR products having a length of at least 2 kb and comprising at least one attenuation locus are generated" herein means that the virus nucleic acids of a) or the double-stranded DNA templates of step b) are incubated with suitable primers (forward and reverse primers), nucleotides and a template-dependent DNA polymerase such that a PCR product of at least 2kb is generated, wherein the PCR product comprises one or more of the attenuation loci of the virus. The reaction may be carried out in an automated thermal cycler.

The term "primer" as used herein denotes an oligonucleotide that acts as an initiation point of nucleotide synthesis under conditions in which synthesis of a primer extension product complementary to a nucleic acid strand is induced. The primers may be generated such that they are able to bind to the sequence to be reverse transcribed or amplified. The primer may comprise from 5 to 50 nucleotides, preferably, 8 to 30 nucleotides; more preferably the primer comprises 10 to 20 nucleotides. The primer sequences are complementary to at least a portion of the sequence to be reverse transcribed or amplified. Preferably, the primer sequences are fully complementary to a sequence within the sequence to be reverse transcribed or amplified. Methods for the design of sequence specific primers and probes are known in the art.

Preferably, the PCR product has a length of at least 3kb, and more preferably 5kb length. Preferably, the PCR product comprises two attenuation loci. More preferably, the PCR product comprises three attenuation loci. For TDV, the PCR product preferably comprises the positions 57, 2579 and/or 5270 of the TDV2 sequence (SEQ ID NO:23).

Preferably, the PCR amplification reaction of step c) comprises at least 10 PCR cycles using a DNA polymerase, suitable forward and reverse primers and nucleotides. More preferably, 10 to 50 cycles, even more preferred 10 to 30 cycles are used. For Dengue virus PCR product amplification, the PCR amplification reaction comprises the use of a first primer pair having the nucleotide sequence of SEQ ID NO: 9 (5F) and a second primer having the nucleotide sequence of SEQ ID NO: 6 (3R). It is also preferred that the PCR amplification reaction includes a denaturing step at a temperature from about 90°C to about 99°C, an annealing step at a temperature from about 45°C to about 75°C, and an extension step at a temperature from about 60°C to about 75°C, more preferably denaturing is performed at about 95°C, annealing is performed at 68°C with a 1°C drop for each cycle over 10 cycles and extension is performed at 68°C.

Preferably, after the amplification step c) and prior to the sequencing step d) the method additionally comprises the steps of:
i) end repairing of the PCR products;
ii) optionally, purifying the end repaired PCR product; and
iii) connecting the two complementary strands of the PCR product prepared in step c) with a linking oligonucleotide, wherein the 3' end of one complementary strand is linked to the 5' end of the other complementary strand via the linking oligonucleotide in each double stranded nucleic acid fragment, whereby the linking oligonucleotide provides a single stranded portion of the resulting linked nucleic acid fragments.

End repairing of the PCR products is intended to generate blunt-end PCR products for the subsequent ligation of adaptors for SMRT sequencing. End repairing can be achieved by the use of suitable DNA polymerases such as the T4 DNA polymerase (Fast DNA End Repair Kit from Thermofischer) and nucleotides.

"Connecting the two complementary strands of the PCR product prepared in step c) with a linking oligonucleotide" herein means that by ligating of hairpin adaptors to both ends of the above double-stranded DNA product a closed, single-stranded circular DNA, the so-called SMRTbell, is created. Suitable hairpin adaptors are available from Pacific Biosciences (SMRTbell® Template Prep Kit). The hairpin adapator is linked to the blunt-ended PCR product using a suitable ligase.

Preferably, prior to step d) the amplified PCR products are purified. Methods for purifying of PCR products are known in the art. Suitable methods include the separation on an agarose gel or purification by column or beads.

"Single-molecule real-time (SMRT) sequencing" of step d) herein means the sequencing techniques and platforms provided by the company Pacific Biosciences. SMRT sequencing preferably includes the PacBio RS and RSII platform and the PacBio Sequel platform, most preferably SMRT sequencing is performed using the PacBio RSII platform. When a sample with the SMRTbell structure is loaded to a chip called a SMRTcell, a SMRTbell diffuses into a sequencing unit called a zero-mode waveguide (ZMW), which provides the smallest available volume for light detection. The ZMW is a structure that creates an illuminated observation volume that is small enough to observe only a single nucleotide of DNA being incorporated by DNA polymerase.

For carrying out the SMRT sequencing to this SMRT cell a primer complementary to the hairpin adaptor is added with a mixture of mononucleotides under suitable conditions. Each of the four mononucleotide bases is attached to one of four different fluorescent dyes. When a nucleotide is incorporated by the DNA polymerase, the fluorescent tag is cleaved off and diffuses out of the observation area of the ZMW where its fluorescence is no longer observable. A detector detects the fluorescent signal of the nucleotide incorporation, and the base call, i.e. the correlation of the base with the fluorescent signal, is made according to the corresponding fluorescence of the dye.

In order to reduce the sequence error rate of SMRT sequencing, a preferably high number of sequence reads is used for data analysis. More preferably, at least 10³, more preferably, 10⁴, most preferred 5x10⁴ reads/run are used. The higher the number of reads confirming a particular sequence, the higher is the statistical reliability of this given sequence. Reads below 10 are considered as unreliable.

SMRT sequencing differs from second-generation sequencing (SGS) technologies such as those provided by the companies Illumina (Miniseq, MiSeq, NextSeq or HiSeq) or Ion Torrent (PGM, S5, Proton), and Roche (454, pyrosequencing). The SGS technologies are characterized by much shorter read length of up to 400 bp, while having a much lower sequence error rate than SMRT sequencing. Quantifying low-frequency revertants in oral poliovirus vaccine was performed using SGS technology, in particular MiSeq System (Illumina) or HiSeq 1500 System (Illumina), and the accuracy was shown to be comparable with the prior art MAPREC (mutant analysis by polymerase chain reaction and restriction enzyme cleavage) assay (Sarcey et al., J. Virol. Methods 246 (2017, 75-80). In view of the short read-lengths SGS technologies do not provide an individual sequence of a large insert such as 1 kb or more, but rather for such inserts the SGS technologies provide an average sequence of different polynucleotides.

The wild-type virus sequences for different viruses are available from public databases are e.g. hosted by NCBI such as Entrez (ww.ncbi.nlm.nih.gov). The wild-type virus sequence of Dengue virus serotype 2 is set forth as SEQ ID NO:23. This wild-type sequence has nucleotide C at position 57, nucleotide G at position 2579 and/or nucleotide A on position 5270.

"Comparing at least one nucleotide at said at least one attenuation locus in said PCR products with the corresponding nucleotide in the wild-type virus sequence" herein means a comparison of the obtained sequence of the PCR products with the wild-type sequence at the attenuation locus/loci. The comparison can be performed manually or using a computer program. If the obtained sequence of the PCR products has the same nucleotide at the attenuation locus as the wild-type, this is indicative of the presence of a revertant in the sample. If the obtained sequence of the PCR products has the same nucleotide at the attenuation locus as the attenuated virus, this is indicative that no revertant virus is present in said sample.

In one embodiment, if the attenuated virus is TDV, at least one nucleotide at said at least one attenuation locus in said PCR products is compared with the corresponding nucleotide in the wild-type virus sequence, preferably at least one nucleotide at two attenuation loci in said PCR products is compared with the corresponding nucleotide in the wild-type virus sequence and more preferably at least one nucleotide at three attenuation loci in said PCR products is compared with the corresponding nucleotide in the wild-type virus sequence.

In one embodiment, if the attenuated virus is TDV, at least one nucleotide at at least one attenuation locus selected from nucleotides corresponding to nucleotides 57, 2579 and 5270 in the nucleic acid sequence according to SEQ ID No. 23 in said PCR products is compared with the corresponding nucleotide in the wild-type virus sequence. Preferably at least one nucleotide at two attenuation loci selected from nucleotides corresponding to nucleotides 57, 2579 and 5270 in the nucleic acid sequence according to SEQ ID No. 23 in said PCR products is compared with the corresponding nucleotide in the wild-type virus sequence.

More preferably at least one nucleotide at three attenuation loci at from nucleotides corresponding to nucleotides 57, 2579 and 5270 in the nucleic acid sequence according to SEQ ID No. 23 in said PCR products is compared with the corresponding nucleotide in the wild-type virus sequence.

According to a further aspect of the invention is provided a method for the quantitative analysis of a mixture of virus haplotypes in a virus sample comprising the steps:
a) purifying virus nucleic acids from said sample;
b) preparing double-stranded DNA templates from said virus nucleic acids by using a primer comprising a unique molecular identifier (UMI) sequence such that said double-stranded DNA templates comprise the UMI sequence;
c) amplifying said double-stranded DNA templates by polymerase chain reaction (PCR) such that PCR products having a length of at least 2kb and comprising a unique molecular identifier (UMI) sequence are generated;
d) sequencing said PCR products by single-molecule real-time (SMRT) sequencing; and
e) determining the relative amounts of said PCR products by analyzing the UMI count and the Read count thereby the virus haplotypes contained in the sample.

The embodiments for the virus and the sample are as defined above. A "virus haplotype" relates to the entire nucleotide sequence of a given virus. A virus haplotype may be defined as the combination of nucleotides at particular loci such as the attenuation loci. For example, the Dengue virus haplotype T:57-A:2579-T:5270 is different from the haplotype T:57-A:2579-C:5270 by the difference in nucleotide 5270.

"Unique molecular identifiers (UMIs)" are sequences of nucleotides applied to or identified in DNA molecules that may be used to distinguish individual DNA molecules from one another (see, e.g., Kivioja, Nature Methods 9, 72-74 (2012)). UMIs may be sequenced along with the DNA molecules with which they are associated to determine whether the read sequences are those of one source DNA molecule or another. The term "UMI" is used herein to refer to both the sequence information of a polynucleotide and the physical polynucleotide per se. The term "UMI" includes bar codes which are commonly used to distinguish reads of one sample from reads of other samples.

Commonly, multiple copies of a single source molecule are sequenced.

The source molecule for the sequencing may be PCR amplified before delivery to a flow cell. Each molecule in a PCR-amplified cluster is derived from the same source DNA molecule, but is separately sequenced. For error correction and other purposes, it can be important to determine that all reads from a single cluster are identified as deriving from the same source molecule. UMIs allow this grouping. A DNA molecule that is copied by amplification or otherwise to produce multiple copies of the DNA molecule is referred to as a source DNA molecule.

UMIs may be applied to or identified in individual DNA molecules. In some embodiments, the UMIs may be applied to the DNA molecules by methods that physically link or bind the UMIs to the DNA molecules, e.g., by ligation or transposition through polymerase, endonuclease, transposases, etc. These "applied" UMIs are therefore also referred to as physical UMIs. In some contexts, they may also be referred to as exogenous UMIs. The UMIs identified within source DNA molecules are referred to as virtual UMIs. In some context, virtual UMIs may also be referred to as endogenous UMIs.

Physical UMIs may be defined in many ways. For example, they may be random, pseudorandom or partially random, or non-random nucleotide sequences that are inserted in adapters or otherwise incorporated in source DNA molecules to be sequenced. In some embodiments, the physical UMIs may be so unique that each of them is expected to uniquely identify any given source DNA molecule present in a sample. The collection of adapters is generated, each having a physical UMI, and those adapters are attached to fragments or other source DNA molecules to be sequenced, and each individual sequenced molecule has a UMI that helps to distinguish it from all other fragments. In such embodiments, a very large number of different physical UMIs (e.g., many thousands to millions) may be used to uniquely identify DNA fragments in a sample.

The physical UMI must have a sufficient length to ensure this uniqueness for each and every source DNA molecule. In some embodiments, a less unique molecular identifier can be used in conjunction with other identification techniques to ensure that each source DNA molecule is uniquely identified during the sequencing process. In such embodiments, multiple fragments or adapters may have the same physical UMI. Other information such as alignment location or virtual UMIs may be combined with the physical UMI to uniquely identify reads as being derived from a single source DNA molecule/fragment. In some embodiments, adaptors include physical UMIs limited to a relatively small number of non-random sequences, e.g., 96 non-random sequences. Such physical UMIs are also referred to as non-random UMIs. In some embodiments, the non-random UMIs may be combined with sequence position information and/or virtual UMIs to identify reads attributable to a same source DNA molecule. The identified reads may be collapsed to obtain a consensus sequence that reflects the sequence of the source DNA molecule as described herein.

A "random UMI" may be considered a physical UMI selected as a random sample, from a set of UMIs consisting of all possible different oligonucleotide sequences given one or more sequence lengths. For instance, if each UMI in the set of UMIs has n nucleotides, then the set includes 4ⁿ UMIs having sequences that are different from each other. A random sample selected from the 4ⁿ UMIs constitutes a random UMI.

Conversely, a "non-random UMI" as used herein refers to a physical UMI that is not a random UMI. In some embodiments, available non-random UMIs are predefined for a particular experiment or application. In certain embodiments, rules are used to generate sequences to obtain a non-random UMI. For instance, the sequences of a set may be generated such that the sequences have a particular pattern or patterns. In some embodiments, each sequence differs from every other sequence in the set by a particular number of (e.g., 2, 3, or 4) nucleotides. That is, no non-random UMI sequence can be converted to any other available non-random UMI sequence by replacing fewer than the particular number of nucleotides. In some embodiments, a non-random UMI is selected from a set of UMIs including fewer than all possible UMIs given a particular sequence length. For instance, a nonrandom UMI having 6 nucleotides may be selected from a total of 96 different sequences (instead of a total of 4⁶=4096 possible different sequences). In other embodiments, sequences are not randomly selected from a set. Instead, some sequences are selected with higher probability than other sequences.

The attachment of UMIs can be performed by adding a UMI sequence such as a random sequence to the 5' end of a forward primer or the 3' end of a reverse primer to be used in the PCR amplification reaction. Thereby, the UMI sequence is incorporated into the PCR amplification product.

If the virus nucleic acids are isolated virus RNA, step b) comprises a step of reverse transcription of the isolated virus RNA in the presence of a reverse transcriptase using a reverse primer additionally comprising at its 5'end a UMI sequence. Preferably, in case of Dengue virus, reverse transcription is performed using as reverse primer an oligonucleotide having the nucleotide sequence of SEQ ID NO:22 (UMI 3R) at a temperature in the range from about 45°C to about 65° C, preferably at a temperature in the range from about 50°C to about 55°C.

Alternatively, the UMI sequence can be incorporated into the forward or reverse primer at the PCR amplification of step c).

Preferably, the PCR amplification of step c) includes a PCR amplification reaction comprising at least 10 PCR cycles using a DNA polymerase and forward and reverse primers. More preferably, 10 to 50 PCR cycles, even more preferred 10 to 30 PCR cycles are performed. In case of Dengue virus, the PCR amplification reaction comprises the use of a first primer having the nucleotide sequence of SEQ ID NO:9 (5F) and a second primer having the nucleotide sequence of SEQ ID NO:21 (Primer IIA).

Preferably, the PCR amplification reaction of step c) includes a denaturing step at a temperature from about 90°C to about 99°C, an annealing step at a temperature from about 45°C to about 75°C, and an extension step at a temperature from about 60°C to about 75°C. Preferably, after the amplification step c) and prior to the sequencing step d) the method additionally comprises the steps of:
i) end repairing of the PCR products;
ii) optionally, purifying the end repaired PCR product; and
iii) connecting the two complementary strands of the PCR product prepared in step c) with a linking oligonucleotide, wherein the 3' end of one complementary strand is linked to the 5' end of the other complementary strand via the linking oligonucleotide in each double stranded nucleic acid fragment, whereby the linking oligonucleotide provides a single stranded portion of the resulting linked nucleic acid fragments.

Embodiments of steps c) and d) are as defined above.

"Read count" represents the number of reads. It is a category of quantification scheme commonly employed in single molecule sequencing. Although the read count-based scheme is similar to the common approaches used for bulk RNA-sequencing, the miniscule quantity of transcripts captured from a single cell requires cDNA amplification for library construction; this inevitably results in large amplification bias. Thus, if a truncated or erroneous cDNA fragment is generated which binds stronger to the used primer pair, the truncated or erroneous cDNA fragment will be amplified in a higher amount compared to the correct DNA fragment. "UMI count" relates to number of the uniquely mapped reads only. To mitigate the bias in read counts, several recent scRNA-sequencing protocols have employed an additional step in which individual transcripts are modified with unique molecular identifiers (UMIs) before amplification, resulting in a more accurate quantification of the transcript count. The UMI count is determined by considering only the Read count of sequences which contain a unique UMI sequence.

According to a preferred embodiment the method of the present invention comprises prior to step d) the steps:
i) determining the sequence of the PCR products at at least one attenuation locus separately by Sanger sequencing;
ii) comparing at least one nucleotide at said at least one attenuation locus with the wild-type virus sequence; and
iii) if the wild-type sequence is determined at at least one attenuation locus, sequencing said PCR products by SMRT sequencing of step d) is carried out.

Sanger sequencing, i.e. a method of DNA sequencing based on the selective incorporation of chain-terminating dideoxynucleotides by DNA polymerase during *in vitro* DNA replication is significantly less expensive compared to SMRT sequencing. If the Sanger sequencing at the individual attenuation loci indicates the presence of a revertant virus, SMRT sequencing of the entire virus nucleic acid molecule is conducted to discriminate single, double and triple revertants at the attenuation loci.

A suitable primer pair for Sanger sequencing of the first attenuation locus at position 57 of the TDV2 nucleotide sequence set forth as SEQ ID NO:23 is the forward primer having the nucleotide sequence of SEQ ID NO: 13 and the reverse primer of SEQ ID NO: 17. A suitable primer pair for Sanger sequencing of the second attenuation locus at position 2579 is the forward primer having the nucleotide sequence of SEQ ID NO: 14 and the reverse primer of SEQ ID NO: 18. Suitable primer pairs for Sanger sequencing of the third attenuation locus at position 5270 are forward primers having a nucleotide sequence of SEQ ID Nos: 15 or 16, and as reverse primers having a nucleotide sequence of SEQ ID Nos: 19 or 20.

According to a further aspect the present invention provides the use of the method according to the first aspect in the quality control of vaccines containing live, attenuated virus. For quality control of vaccine samples, it is of particular importance to detect the existence of revertants in the vaccine sample. It is readily apparent that dengue virus having a reversion at each of the three attenuation loci (triple revertant) has the wild-type replication capacity and may therefore be virulent. However, a vaccine composition comprising a double revertant having a reversion at two of the attenuation loci and a monorevertant having a reversion at the third attenuation locus may also pose a risk in view of the possibility of recombination events between the double revertant and the single revertant which may lead to increased virulence.

According to a further aspect the present invention provides the use of the method according to the second aspect in the diagnosis of a virus infection in a patient sample. The method according to the second aspect may be used for the diagnosis of a virus infection in a virus infected patient. The method also enables the detection of co-infection with different subtypes of viruses such as different dengue serotypes. This may provide advantages in the personalized treatment of the infected patients.

According to a further aspect of the present invention a kit is provided for detecting the presence of at least one revertant virus in a sample containing an attenuated dengue virus comprising a first primer selected from an oligonucleotide having the nucleotide sequence of SEQ ID NO: 1, SEQ ID NO: 3, SEQ ID NO: 5, SEQ ID NO: 7, SEQ ID NO:9 and SEQ ID NO:11, and comprising a second primer selected from an oligonucleotide having the nucleotide sequence of SEQ ID NO: 2, SEQ ID NO: 4, SEQ ID NO: 6, SEQ ID NO:8, SEQ ID NO: 10 and SEQ ID NO: 12. Preferably, the first primer comprises the nucleotide sequence of SEQ ID NO:9 (5F) and the second primer comprises the nucleotide sequence of SEQ ID NO:6 (3R). More preferably, the first primer consists of the nucleotide sequence of SEQ ID NO:9 and the second primer consists of the nucleotide sequence of SEQ ID NO:6.

The kit may further include a suitable reverse transcriptase, nucleotides and suitable buffers known in the art.

According to a further aspect of the present invention a kit is provided for the quantitative analysis of a mixture of virus haplotypes in a dengue virus sample comprising a first primer comprising the nucleotide sequence of SEQ ID NO:9 (5F) and a second primer comprising the nucleotide sequence of SEQ ID NO:21 (Primer IIA). Preferably the first primer consists of the nucleotide sequence of SEQ ID NO:9 and the second primer consists of the nucleotide sequence of SEQ ID NO:21.

The kit may further include a suitable reverse transcriptase, nucleotides and suitable buffers known in the art.

The invention is further described in the following examples which are solely for the purpose of illustrating specific embodiments of the invention, and are also not to be construed as limiting the scope of the invention in any way.

### List of Embodiments

1. A method for detecting the presence of at least one revertant virus in a sample containing an attenuated virus comprising the steps:
   a) purifying virus nucleic acids from said sample;
   b) optionally, preparing double-stranded DNA templates from said virus nucleic acids;
   c) amplifying said viral nucleic acids of a) or said double-stranded DNA templates of b) by polymerase chain reaction (PCR) such that PCR products having a length of at least 2 kb and comprising at least one attenuation locus of said attenuated virus are generated;
   d) sequencing said PCR products by single-molecule real-time (SMRT) sequencing; and
   e) comparing at least one nucleotide at said at least one attenuation locus in said PCR products with the corresponding nucleotide in the wild-type virus sequence, wherein the presence of the wild-type nucleotide at said at least one attenuation locus is indicative of the presence of a revertant virus in said sample.
2. The method of embodiment 1, wherein said virus is an RNA virus, preferably wherein the RNA virus is selected from dengue virus, poliovirus, rubella virus, measles virus, yellow fever virus, mumps virus and influenza virus.
3. The method of embodiment 1, wherein said virus is a DNA virus, preferably wherein the DNA virus is varicella zoster virus, vaccinia virus, smallpox virus, Herpes simplex virus and chikungunya virus.
4. The method of any one of embodiments 1 to 3, wherein said PCR products have a length of at least 3 kb, preferably of at least 5 kb.
5. The method of any one of embodiments 1 to 4, wherein said PCR products comprise at least two attenuation loci, preferably at least three attenuation loci.
6. The method of any one of embodiments 1, 2, 4 or 5, wherein said attenuated virus is a live, attenuated dengue virus and said at least one attenuation locus is selected from positions 57, 2579 and/or 5270 of the nucleotide sequence of dengue virus type 2 according to SEQ ID NO: 23.
7. The method of any one of embodiments 1 to 6, wherein prior to step a) the live, attenuated virus is grown in Vero cells.
8. The method of any one of embodiments 1, 2 or 4 to 7, wherein the virus nucleic acids are isolated virus RNA and step b) comprises a step of reverse transcription of the isolated virus RNA in the presence of a reverse transcriptase using a reverse primer.
9. The method of embodiment 8, wherein the reverse transcription is performed using as reverse primer an oligonucleotide comprising the nucleotide sequence of SEQ ID NO:6 (3R) at a temperature in the range from about 45°C to about 65° C, preferably at a temperature in the range from about 50°C to about 55°C, preferably the oligonucleotide consists of the nucleotide sequence of SEQ ID NO:6.
10. The method of any one of embodiments 1 to 9, wherein step c) includes a PCR amplification reaction comprising at least 10 PCR cycles using a DNA polymerase and forward and reverse primers.
11. The method of embodiment 10, wherein the PCR amplification reaction comprises the use of a first primer comprising the nucleotide sequence of SEQ ID NO:6 (3R) and a second primer comprising the nucleotide sequence of SEQ ID NO:9 (5F), preferably the first primer consists of the nucleotide sequence of SEQ ID NO:6 and the second primer consists of the nucleotide sequence of SEQ ID NO:9.
12. The method of embodiment 10 or 11, wherein the PCR amplification reaction includes a denaturing step at a temperature from about 90°C to about 99°C, an annealing step at a temperature from about 45°C to about 75°C, and an extension step at a temperature from about 60°C to about 75°C.
13. The method of any one of embodiments 1 to 12, wherein the amplified PCR products obtained in step c) are purified prior to step d).
14. The method of any one of embodiments 1 to 13, wherein prior to step d) the amplified PCR products are provided as a library suitable for SMRT sequencing.
15. The method of embodiment 14, wherein prior to step d) the method additionally comprises:
   i) end repairing of the PCR products;
   ii) optionally, purifying the end repaired PCR product; and
   iii) connecting the two complementary strands of the PCR product prepared in step c) with a linking oligonucleotide, wherein the 3' end of one complementary strand is linked to the 5' end of the other complementary strand via the linking oligonucleotide in each double stranded nucleic acid fragment, whereby the linking oligonucleotide provides a single stranded portion of the resulting linked nucleic acid fragments.
16. A method for the quantitative analysis of a mixture of virus haplotypes in a virus sample comprising the steps of:
   a) purifying virus nucleic acids from said sample;
   b) preparing double-stranded DNA templates from said virus nucleic acids by using a primer comprising a unique molecular identifier (UMI) sequence such that said double-stranded DNA templates comprise the UMI sequence;
   c) amplifying said double-stranded DNA templates by polymerase chain reaction (PCR) such that PCR products having a length of at least 2kb and comprising a unique molecular identifier (UMI) sequence are generated;
   d) sequencing said PCR products by single-molecule real-time (SMRT) sequencing; and
   e) determining the relative amounts of said PCR products by analysing the UMI count and the Read count, thereby quantifying the virus haplotypes contained in the sample.
17. The method of embodiment 16, wherein any one of the virus haplotypes is present in the virus sample in a quantity of less than 10 %, preferably less than 1 %, more preferably less than 0.2 % based on the total amount of virus in the sample.
18. The method of embodiment 16, wherein the virus is an RNA virus, preferably wherein the RNA virus is selected from dengue virus, poliovirus, rubella virus, measles virus, yellow fever virus, mumps virus and influenza virus.
19. The method of embodiment 16, wherein the virus is a DNA virus, preferably wherein the DNA virus is varicella zoster virus.
20. The method of embodiment 16 or 17, wherein said virus is a live, attenuated dengue virus, preferably a dengue virus comprising at least one attenuation locus selected from positions 57, 2579 and/or 5270 of the nucleotide sequence of dengue virus type 2 according to SEQ ID NO: 23.
21. The method of any one of embodiments 16 to 19, wherein said PCR products have a length of at least 3 kb, preferably of at least 5 kb.
22. The method of any one of embodiments 16, 17, or 20, wherein the virus nucleic acids are isolated virus RNA and step b) comprises a step of reverse transcription of the isolated virus RNA in the presence of a reverse transcriptase using a reverse primer additionally comprising at its 5'end a UMI sequence.
23. The method of embodiment 22, wherein the reverse transcription is performed using as reverse primer an oligonucleotide comprising the nucleotide sequence of SEQ ID NO:22 (UMI 3R) at a temperature in the range from about 45°C to about 65° C, preferably the oligonucleotide consists of the nucleotide sequence of SEQ ID NO:22 and the temperature is in the range from about 50°C to about 55°C.
24. The method of any one of embodiments 16 to 23, wherein step c) includes a PCR amplification reaction comprising at least 10 PCR cycles using a DNA polymerase and forward and reverse primers.
25. The method of embodiment 24, wherein the PCR amplification reaction comprises the use of a first primer comprising the nucleotide sequence of SEQ ID NO:21 (Primer IIA) and a second primer comprising the nucleotide sequence of SEQ ID NO: 9 (5F), preferably the first primer consists of the nucleotide sequence of SEQ ID NO:21 and the second primer consists of the nucleotide sequence of SEQ ID NO:9.
26. The method of embodiment 24 or 25, wherein the PCR amplification reaction includes a denaturing step at a temperature from about 90°C to about 99°C, an annealing step at a temperature from about 45°C to about 75°C, and an extension step at a temperature from about 60°C to about 75°C.
27. The method of any one of embodiments 16 to 26, wherein the amplified PCR products obtained in step c) are purified prior to step d).
28. The method of any one of embodiments 16 to 27, wherein prior to step d) the amplified PCR products are provided as a library suitable for SMRT sequencing.
29. The method of embodiment 28, wherein after the amplification step c) and prior to the sequencing step d) the method additionally comprises:
   i) end repairing of the PCR products;
   ii) optionally, purifying the end repaired PCR product; and
   iii) connecting the two complementary strands of the double stranded nucleic acid fragments prepared in step (b) with a linking oligonucleotide, wherein the 3' end of one complementary strand is linked to the 5' end of the other complementary strand via the linking oligonucleotide in each double stranded nucleic acid fragment, whereby the linking oligonucleotide provides a single stranded portion of the resulting linked nucleic acid fragments.
30. The method according to any one of embodiments 1 to 29, wherein said sample is a composition comprising an attenuated virus, or a sample from a patient.
31. The method of any one of embodiments 1 to 30, wherein prior to step d) the method comprises:
   i) determining the sequence of the PCR products at at least one of the attenuation locus separately by Sanger sequencing;
   ii) comparing at least one nucleotide at said at least one attenuation locus with the wild-type virus sequence; and
   iii) if the wild-type sequence is determined at at least one attenuation locus, sequencing said PCR products by SMRT sequencing of step d) is carried out.
32. Use of the method according to any one of embodiments 1 to 15 in the quality control of vaccines containing live, attenuated virus.
33. Use of the method according to any one of embodiments 16 to 31 in the diagnosis of a virus infection in a patient sample.
34. A kit for detecting the presence of at least one revertant virus in a sample containing an attenuated dengue virus comprising a first primer selected from an oligonucleotide having the nucleotide sequence of SEQ ID NO: 1, SEQ ID NO: 3, SEQ ID NO: 5, SEQ ID NO: 7, SEQ ID NO:9 and SEQ ID NO:11, and comprising a second primer selected from an oligonucleotide having the nucleotide sequence of SEQ ID NO: 2, SEQ ID NO: 4, SEQ ID NO: 6, SEQ ID NO:8, SEQ ID NO: 10 and SEQ ID NO: 12, preferably the first primer comprises the nucleotide sequence of SEQ ID NO: 9 (5F) and the second primer comprises the nucleotide sequence of SEQ ID NO: 6 (3R), more preferably, the first primer consists of the nucleotide sequence of SEQ ID NO:9 and the second primer consists of the nucleotide sequence of SEQ ID NO:6.
35. A kit for the quantitative analysis of a mixture of virus haplotypes in a dengue virus sample comprising a first primer comprising the nucleotide sequence of SEQ ID NO:21 (Primer IIA) and a second primer comprising the nucleotide sequence of SEQ ID NO: 9 (5F), preferably the first primer consists of the nucleotide sequence of SEQ ID NO:21 and the second primer consists of the nucleotide sequence of SEQ ID NO:9.

### Examples

### Material and Methods

### Growth and Infection of Vero cells

Vero WHO cells were seeded in T25 cm² flasks at 1.5e⁶ cells/flask and grown overnight to 80-90% confluency by incubating at 37°C with 5% CO₂ tension. Cells were observed for health and confluency under an inverted microscope before the start of the infection experiment.

TDV-2 virus was diluted in assay medium DMEM containing 1% FBS and 1% Penicillin/Streptomycin (Hyclone, Cat # SV30010). Vero cells were infected with TDV-2 at 0.1MOI and allowed virus adsorption for 1 hour at 37°C 5% CO₂ incubator. Upon completion of incubation, inoculum was aspirated and cells were replenished with 6ml of fresh assay medium. The flasks were allowed to continue incubate at this temperature for additional 5-7 days. Medium was changed on day 5 and virus was harvested on day 7 by collecting and spinning the cell culture supernatant at 10,000 x g for 10 minutes in a 4°C cooled centrifuge.

### Viral RNA Extraction

140µl of TDV2/Vero WHO viral cell culture supernatant was subjected to RNA extraction using 3 different commercial kits. The extraction was carried out according to the instructions came with the kits. Qiagen QIAamp Viral RNA Mini Kit (Lot# 145051122), Omega Viral RNA extraction kit and Roche's High Pure RNA Isolation kit as per the instructions of the manufacturers without addition of carrier RNA. The purified RNA yield was measured on a Nanodrop spectrophotometer.

### Optimization of Pacbio Sequencing for TDV analysis

### Process Summary: Flow Chart

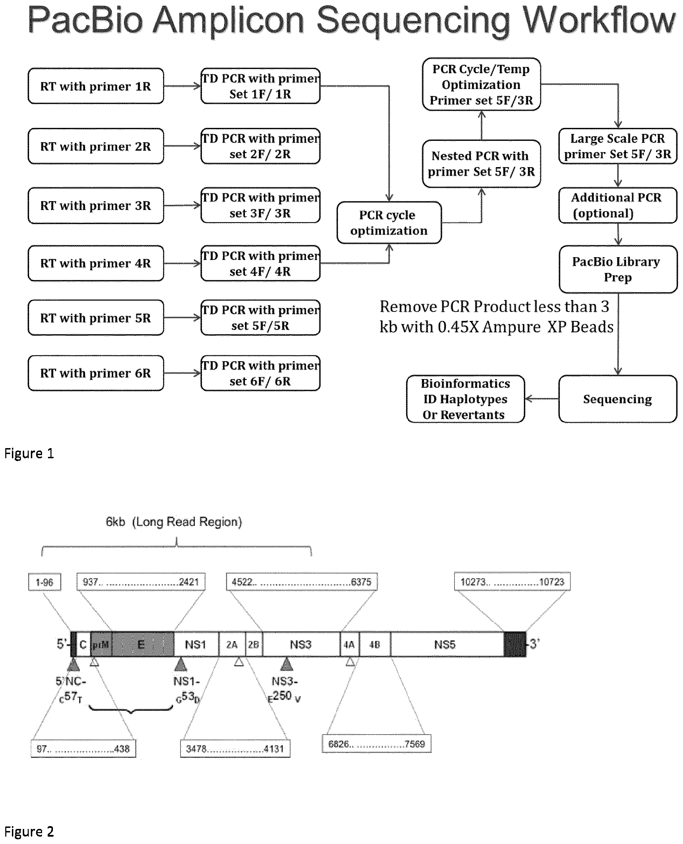

The pacbio sequencing work flow has been described in Figure 1. Reverse transcription (RT) was first performed with selected primers followed by PCR. The PCR conditions were then optimized. The PCR products was enriched by nested PCR and further amplifications. The PCR products were treated with a kit for the production of pacbio library. Smaller products were removed using Ampure beads. Then SMRT sequencing was performed and data was analyzed using bioinformatic tools.

### Primer Selection

The regions surrounding the variants at positions 57 and 5270 were analyzed using the RNA folding software (http://unafold.rna.albany.edu/?q=mfold) to identify regions having minimal secondary structure. Segments were chosen having unpaired regions and six primer pairs were chosen in these segments using the PRIMER3 server at NCBI. Since only 57 bp are available at the 5' end (Figure 2) some of these primers overlap with one another (Table 1).

### List of primers used for RT-PCR optimization

**Table 1: Primer sequences and characteristics. Note that the reverse primer is also the primer for reverse transcription. The primers are in pairs.**

| **Name** | **Dir.** | **Sequence** | **Start** | **Stop** | **Tm** | **Product** |
|---|---|---|---|---|---|---|
| 1F | F | AAGACAGATTCTTTGAGGGAGCTA | 26 | 49 | 59 | 5368 |
| 1R | R | GGTCTGTGAAATGGGCTTCG | 5393 | 5374 | 59 | 5368 |
| 2F | F | GACAGATTCTTTGAGGGAGCTAA | 28 | 50 | 58 | 5367 |
| 2R | R | GGGTCTGTGAAATGGGCTTC | 5394 | 5375 | 59 | 5367 |
| 3F | F | ACAGATTCTTTGAGGGAGCTAAG | 29 | 51 | 58 | 5364 |
| 3R | R | GTCTGTGAAATGGGCTTCGTC | 5392 | 5372 | 60 | 5364 |
| 4F | F | AGACAGATTCTTTGAGGGAGCTAAG | 27 | 51 | 60 | 5366 |
| 4R | R | GTCTGTGAAATGGGCTTCGT | 5392 | 5373 | 58 | 5366 |
| 5F | F | GACAGATTCTTTGAGGGAGCTAAG | 28 | 51 | 59 | 5373 |
| 5R | R | CTTGCTGGGTCTGTGAAATGG | 5400 | 5380 | 59 | 5373 |
| 6F | F | AAGACAGATTCTTTGAGGGAGCTAA | 26 | 50 | 60 | 5370 |
| 6R | R | TGGGTCTGTGAAATGGGCTTC | 5395 | 5375 | 61 | 5370 |

**Table 2: Correlation of Primer Sequences with SEQ ID numbers.**

| **Sequence** | **Name** |
|---|---|
| AAGACAGATTCTTTGAGGGAGCTA | 1F (SEQ ID NO:1) |
| GGTCTGTGAAATGGGCTTCG | 1R (SEQ ID NO:2) |
| GACAGATTCTTTGAGGGAGCTAA | 2F (SEQ ID NO:3) |
| GGGTCTGTGAAATGGGCTTC | 2R (SEQ ID NO:4) |
| ACAGATTCTTTGAGGGAGCTAAG | 3F (SEQ ID NO:5) |
| GTCTGTGAAATGGGCTTCGTC | 3R (SEQ ID NO:6) |
| AGACAGATTCTTTGAGGGAGCTAAG | 4F (SEQ ID NO:7) |
| GTCTGTGAAATGGGCTTCGT | 4R (SEQ ID NO:8) |
| GACAGATTCTTTGAGGGAGCTAAG | 5F (SEQ ID NO:9) |
| CTTGCTGGGTCTGTGAAATGG | 5R (SEQ ID NO:10) |
| AAGACAGATTCTTTGAGGGAGCTAA | 6F (SEQ ID NO:11) |
| TGGGTCTGTGAAATGGGCTTC | 6R (SEQ ID NO:12) |

Primers were obtained from IDT DNA (www.idtdna.com). The reverse primer in each case was used as the primer for reverse transcriptase in the first strand cDNA reaction. The pair was then used for PCR.

### RT- PCR optimization

Reverse transcription was carried out on 6.5 µL (139.1 ng) of the TDV2 viral RNA with the reverse primers (Table 1). The six primer pairs were used in touchdown PCR reactions. Touchdown PCR was done as follows. 95 °C for 30 sec., annealing at 66 °C for 20 sec. with a 1 degree drop each cycle for 10 cycles (i.e., cycle 1 - 66°C, cycle 2 - 65°C, cycle 3 - 64°C etc.) and extension at 68 °C for 6 min. An additional 25 cycles were run at 95 °C for 30 sec., annealing at 53 °C for 20 sec. and extension at 68 C for 6 minutes. RT-PCR products were run on 1% agarose gels in 1x TAE buffer.

The two best-performing primer pairs (1 and 4) were then used with nested PCR primers (5F and 3R, from the same set in Table 1) and optimized for number of cycles. Primer pair 1 (1F/R) with fewer non-specific bands was taken forward to annealing temperature optimization. Five temperatures were tested over a range of PCR cycles. The optimum of 11 cycles was chosen and a 0.45X Ampure XP bead purification was used to remove non-specific amplification products less than 3 kilobases.

### PacBio Sequencing and Variant Analysis

The material from optimized RT-PCR reaction was used to construct a PacBio sequencing library. One SMRTcell was sequenced, producing 366 Mbp of data and 12,467 reads of insert. The reads were aligned to the dengue virus reference genome (GenBank accession U87411) using BLASR and variants were called with QUIVER. The data are summarized in the Result section.

### IDENTIFICATION OF TDV2 REVERTANTS

### SANGER SEQUENCING to confirm the identity of the revertants

Four revertants (see Table 3 below) were amplified in Vero WHO cells and viral RNA was extracted using Omega Kit. Sanger sequencing was conducted to confirm identity of these TDV revertants.

**Table 3: List of TDV2 revertants**

| Revertant | Date produced | Reversion Site | TDV | WT |
|---|---|---|---|---|
| ✔ D2/IC **P1** | 8/12/1998 | NS1-2579 | A | **G** |
| ✔ D2/IC **P3** | 6/9/2004 | NS3-5270 | T | **A** |
| ✔ D2/IC **P5** | 8/12/1998 | 5'NC-57 | T | **C** |
| ✔ D2/IC **P51** | 8/12/1998 | 5'NC-57 | T | **C** |
| | | NS1-2579 | A | **G** |

### RT-PCR Optimization

Titan One tube RT-PCR kit (Roche) along with multiple sets of primers (not all shown here) and TDV2 viral RNA was used to optimize RT and PCR conditions to produce a single large product of around 5.3 kb size covering three attenuation loci including nt57, nt2579 and nt5270. Finally, a best pair of primers (shown below) that produced no non-specific products was selected.
D2-1 (Forward): AGTTGTTAGTCTACGTGGACCGAC (SEQ ID NO: 13)
cD2-5358 (Reverse): GAAATGGGCTTCGTCCATGATAATCAGG (SEQ ID NO:20)

Optimized RT-PCR conditions were applied to four TDV2 revertants to amplify 5.3 kb product using 100 ng viral RNA per reaction tube. The RT-PCR was done as follows: 50 °C for 30 sec., 94 °C for 2 minutes (1 cycle), denaturation at 94 °C for 15 sec., annealing at 60 °C for 30 sec. and extension at 68 °C for 5 min (30 cycles) and final extension at 68 °C for 5 minutes (1 cycle). We successfully produced 5.3 kb long products that were subjected to sanger sequencing to identify TDV2 revertants.

### Purification of PCR product

RT-PCR reaction was optimized and PCR products of 5.35 kb were successfully amplified. Two purification kits from Qiagen were used to purify PCR products; Gel extraction and PCR purification kits. Gel extracted kit provided cleaner single band product and was selected for purification of PCR products.

### Sanger Sequencing and Analysis

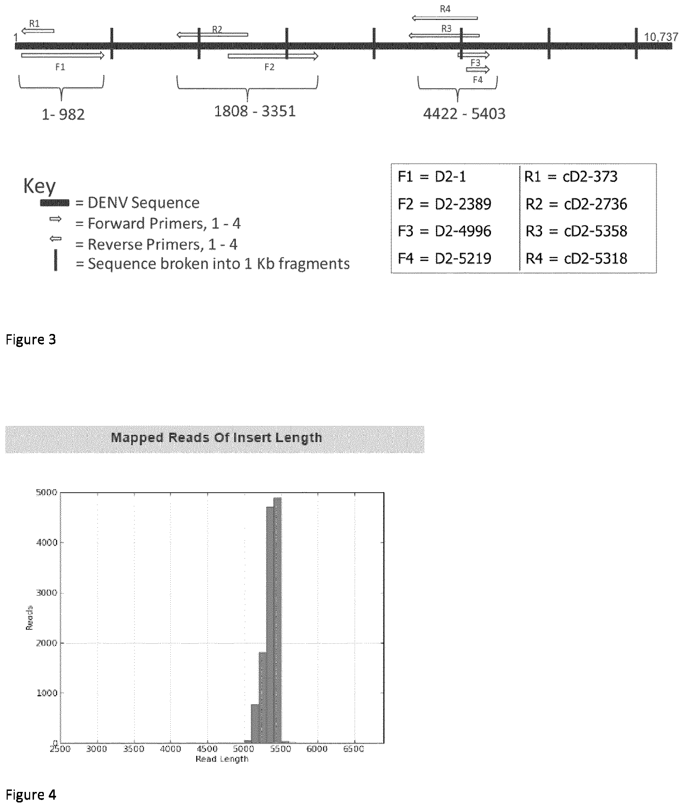

Purified PCR products were examined by Sanger sequencing and sequencing data files were edited and aligned to TDV2 reference sequencing at Takeda. The primers used in Sanger sequencing reactions and the positions of these primers on TDV2 genome covering three attenuation loci of interest can be seen in table and figure of Table 4 and Figure 3, respectively.

### List of primers used for Sanger Sequencing

**Table 4: Primers for Sanger Sequencing**

| **Name** | **Direction** | **Sequence** |
|---|---|---|
| D2-1 (SEQ ID NO:13) | Forward | AGTTGTTAGTCTACGTGGACCGAC |
| D2-2389 (SEQ ID NO:14) | Forward | GTGACACTGTATTTGGGAGTCATGGTGCAG |
| D2-4996 (SEQ ID NO:15) | Forward | GGTAATGGTGTTGTTACAAGGAGTG |
| D2-5219 (SEQ ID NO:16) | Forward | AAGCCCTTAGAGGACTTCCAATAA |
| cD2-373 (SEQ ID NO:17) | Reverse | TCAGCATCCTTCCAATCTCTTTCC |
| cD2-2736 (SEQ ID NO:18) | Reverse | CCGCAGAGATCGTTTTCCTGCCTG |
| cD2-5318 (SEQ ID NO:19) | Reverse | CTAACTGGTGATAGCAGCCTCATGG |
| cD2-5358 (SEQ ID NO:20) | Reverse | GAAATGGGCTTCGTCCATGATAATCAGG |

| Primer conc. = 10uM in Tris buffer, pH 7.5 | | |
|---|---|---|
| vRNA per Reaction = 100ng in Tris buffer | | |
| **Primer** | **Reversion Region** | **Reversion nt site** |
| D2-1 or cD2-373 | 5' NCR | 57 |
| D2-2389 or cD2-2736 | NS1 | 2579 |
| D2-4996 or cD2-5318 | NS3 | 5270 |
| D2-5219 or cD2-5358 | NS3 | 5270 |

### PACBIO SEQUENCING AND VARIANT ANALYSIS

### RT-PCR to amplify PCR product

The optimized RT-PCR procedure was used to amplify cDNA from four TDV2 revertants viral RNA using gene specific primers (GSP). Reverse transcription was carried out on 6.5 µL of viral RNA with 3R reverse primer following by nested PCR using two best performing primer pairs (5F and 3R) at 25, 30, 35 and 40 PCR cycles. The optimum of 30 cycles was chosen and a 0.45X Ampure XP bead purification was used to remove non-specific amplification products less than 3 kilobases.

### PacBio Sequencing and Variant Analysis

The materials from RT-PCR reactions were used to construct PacBio sequencing libraries.

### Materials and Methods for Pacbio's Amplicon Template Preparation and Sequencing Protocol:

### Materials:

- SMRTbell™ Template Prep Kit. Pacbio 100-259-100
- DNA/Polymerase Binding Kit. Pacbio 100-372-700
- MagBead Kit. Pacbio catalog number 100-676-500
- DNA Sequencing Reagent. Pacbio 100-612-400
- DNA Internal Control Complex. Pacbio 100-364-600
- SMRT® Cells. Pacbio 100-171-800
- Pre-washed AMPure XP Beads. Beckman Coulter A63881
- Qiagen Buffer EB. Qiagen 19086
- Magnetic rack. ThermoFisher Scientific 12321
- DynaMag™-96 Bottom Magnet. ThermoFisher Scientific 12332D
- Eppendorf™ DNA LoBind Microcentrifuge Tubes, Eppendorf 022431021
- VWR® PCR 8-Well Tube Strips, VWR 20170-004
- Qubit™ dsDNA BR Assay Kit, ThermoFisher Scientific Q32850
- Agilent DNA 12000 Kit, Agilent 5067-1508
- Hard-Shell PCR Plates 96-well, thin wall, BioRad HSP9655

### Pre-wash AMPure XP Beads: (Use washed AMPure XP beads for all library prep steps)

1. Thoroughly re-suspend AMPure XP beads.
2. Pipette 500 µl of AMPure XP beads into a 1.5 ml centrifuge tube.
3. Spin for 1 minute at max speed in a bench top microcentrifuge.
4. Place the tube on a magnetic rack until the supernatant clears.
5. Transfer the supernatant to another 1.5 ml centrifuge tube and save for later.
6. Add 1 ml of molecular biology grade water to the beads and vortex to re-suspend the beads completely.
7. Centrifuge the beads for 1 minute at max speed in a bench top microcentrifuge.
8. Place the tube on a magnetic rack until the supernatant clears.
9. Discard the water.
10. Repeat the water wash 4 more times (steps 6-9).
11. Add 1 ml of Qiagen buffer EB to the bead pellet and vortex to completely re-suspend beads.
12. Centrifuge for 1 minute at max speed in a bench top microcentrifuge.
13. Place the bead tube on a magnetic rack until the supernatant clears.
14. Discard the EB buffer.
15. Completely re-suspend the beads in the original AMPure XP supernatant collected in step 5 by vortexing.
16. Store the washed beads at 4 °C for up to 3 months.

### Purify PCR Product:

1. Determine the sample volume of your large-scale PCR reactions. In this case, 3X PCR reactions produced 150 µl of product for each sample. Move the PCR products to new 1.5 ml LoBind microcentrifuge tubes. Add 0.45X volumes (67.5 µl) of AMPure XP beads to each sample.
2. Mix the bead/DNA solution thoroughly.
3. Quickly spin down the tubes to collect the beads.
4. Allow the DNA to bind to the beads by rotating the tubes for 10 minutes at room temperature. Note that the bead/DNA mixing is critical to yield. After rotating, the bead/DNA mixture should appear homogenous. Failure to thoroughly mix the DNA with the bead reagent will result in inefficient DNA binding and reduced sample recoveries.
5. Centrifuge the tubes for 1-5 seconds.
6. Place the tubes in a magnetic bead rack until the beads collect to the side of the tubes and the solution appears clear. The actual time required to collect the beads to the side depends on the volume of beads added.
7. With the tubes still on the magnetic bead rack, slowly pipette off cleared supernatant. Avoid disturbing the bead pellet.
8. Wash the beads by slowly dispensing 1.5 ml of freshly prepared 70% ethanol against the side of the tube opposite the beads. Let the tubes sit for 30 seconds. Do not disturb the bead pellet. After 30 seconds, pipette and discard the ethanol.
9. Repeat step 8 above.
10. Remove residual 70% ethanol and dry the bead pellet. Remove tubes from the magnetic bead rack and spin to pellet beads. Both the beads and any residual 70% ethanol will be at the bottom of the tube. Place the tubes back on magnetic bead rack. Pipette off any remaining 70% ethanol.
11. Check for any remaining droplets in the tubes. If droplets are present, repeat step 10.
12. Remove the tubes from the magnetic bead rack and allow beads to air-dry with the tube caps open for 30 to 60 seconds.
13. Add 39 µl of Qiagen Buffer EB to the beads. Mix until homogenous by vortexing for 1 to 2 minutes at 2000 rpm. Spin the tubes down to pellet the beads and then place the tubes on the magnetic rack until the supernatant clears. Collect 37 µl the eluted sample and place into new 200 µl PCR tubes. Place the sample on ice.

### Repair DNA Damage:

1. Add the following reagents from the SMRTbell™ Template Prep Kit to each sample.

| | |
|---|---|
| DNA Damage Repair Buffer | 5 µl |
| NAD+ | 0.5 µl |
| ATP high | 5 µl |
| dNTP | 0.5 µl |
| DNA Damage Repair Mix | 2 µl |

| | |
|---|---|
| * Make a master mix of DNA Damage Repair Buffer, NAD+, ATP high, and dNTP when preparing more than 1 sample. Add 11 µl of master mix and 2 ul of DNA Damage Repair Mix to each sample. | |

2. Mix the reaction well by flicking the tube.
3. Centrifuge quickly to collect the reaction in the bottom of the tube.
4. Place the sample tubes in a thermacycler and incubate at 37 °C for 1 hr and then at 4 °C for 1 minute. Place the samples on ice.

### Repair Ends:

1. Add the following reagents from the SMRTbell™ Template Prep Kit to each sample.

| | |
|---|---|
| End Repair Mix | 2.5 µl |

2. Mix the reaction well by flicking the sample tubes.
3. Centrifuge briefly to collect samples.
4. Place the samples in a thermocycler and incubate at 25 °C for 5 minutes and then place the samples back on ice. Proceed immediately to the next step and do not allow the reaction to incubate longer than 5 minutes.

### Purify End Repaired Product:

1. Add 0.45X volumes (23.6 µl) of AMPure XP beads to each sample.
2. Mix the bead/DNA solution thoroughly by flicking the tubes.
3. Quickly spin down the tubes to collect the beads.
4. Allow the DNA to bind to the beads by rotating the tubes for 10 minutes at room temperature. Note that the bead/DNA mixing is critical to yield. After rotating, the bead/DNA mixture should appear homogenous. Failure to thoroughly mix the DNA with the bead reagent will result in inefficient DNA binding and reduced sample recoveries.
5. Centrifuge the tubes for 1-5 seconds.
6. Place the tubes in a DynaMag™-96 Bottom Magnet bead rack until the beads collect to the side of the tube and the solution appears clear.
7. With the tubes still on the magnetic bead rack, slowly pipette off cleared supernatant. Avoid disturbing the bead pellet.
8. Wash the beads by slowly dispensing 200 µl of freshly prepared 70% ethanol against the side of the tube opposite the beads. Let the tubes sit for 30 seconds. Do not disturb the bead pellet. After 30 seconds, pipette and discard the ethanol.
9. Repeat step 8 above.
10. Remove residual 70% ethanol and dry the bead pellet. Remove tubes from the magnetic bead rack and spin to pellet beads. Both the beads and any residual 70% ethanol will be at the bottom of the tubes. Place the tubes back on magnetic bead rack. Pipette off any remaining 70% ethanol.
11. Check for any remaining droplets in the tubes. If droplets are present, repeat step 10.
12. Remove the tubes from the magnetic bead rack and allow beads to air-dry with the tube caps open for 30 to 60 seconds.
13. Add 33 µl of Qiagen Buffer EB to the beads. Mix until homogenous by vortexing for 1 to 2 minutes at 2000 rpm. Spin the tubes down to pellet the beads and then place the tubes on the magnetic rack until the supernatant clears. Collect 31 µl of each eluted sample and place into new 200 µl PCR tubes.
14. Place the samples on ice.

### Blunt Ligation:

1. Add the following reagents from the SMRTbell™ Template Prep Kit to each sample. If making a master mix, combine all of the components below except for the Blunt Adapter. Aliquot 7 µl of the master mix to each sample and then add the adapter directly to the mixture.

| | |
|---|---|
| Blunt Adapter | 2 µl |
| Template Prep Buffer | 4 µl |
| ATP Lo | 2 µl |
| Ligase | 1 µl |

2. Mix the reaction by flicking the tubes.
3. Centrifuge the tubes briefly to collect the samples
4. Place the samples in a thermocycler and incubate at 25 °C for 1 hr and then at 65 °C for 10 minutes to inactivate the ligase. Place the samples back on ice.
5. Add 0.5 µl of Exo III and 0.5 µl ExoVII to each sample and incubate the samples at 37°C for 1 hr.
6. You must proceed to the next step.

### Purify SMRTBell Libraries

1. Add 0.45X volumes (18.45 µl) of AMPure XP beads to each sample.
2. Mix the bead/DNA solution thoroughly by flicking the tubes.
3. Quickly spin down the tubes to collect the beads.
4. Allow the DNA to bind to the beads by rotating the tubes for 10 minutes at room temperature. Note that the bead/DNA mixing is critical to yield. After rotating, the bead/DNA mixture should appear homogenous. Failure to thoroughly mix the DNA with the bead reagent will result in inefficient DNA binding and reduced sample recoveries.
5. Centrifuge the tubes for 1-5 seconds.
6. Place the tubes in DynaMag™-96 Bottom Magnet bead rack until the beads collect to the side of the tube and the solution appears clear.
7. With the tubes still on the magnetic bead rack, slowly pipette off cleared supernatant. Avoid disturbing the bead pellet.
8. Wash the beads by slowly dispensing 200 µl of freshly prepared 70% ethanol against the side of the tube opposite the beads. Let the tubes sit for 30 seconds. Do not disturb the bead pellet. After 30 seconds, pipette and discard the ethanol.
9. Repeat step 8 above.
10. Remove residual 70% ethanol and dry the bead pellet. Remove tubes from the magnetic bead rack and spin to pellet beads. Both the beads and any residual 70% ethanol will be at the bottom of the tubes. Place the tubes back on the magnetic bead rack. Pipette off any remaining 70% ethanol.
11. Check for any remaining droplets in the tubes. If droplets are present, repeat step 10.
12. Remove the tubes from the magnetic bead rack and allow beads to air-dry with the tube caps open for 30 to 60 seconds.
13. Add 52 µl of Qiagen Buffer EB to the beads. Mix until homogenous by vortexing for 1 to 2 minutes at 2000 rpm. Spin the tubes down to pellet the beads and then place the tubes on the magnetic rack until the supernatant clears. Collect 50 µl of each eluted sample and place into new 200 µl PCR tubes.
14. Add 0.45X volumes (22.5 µl) of AMPure Xp beads to the elute sample. Repeat steps 2 - 12.
15. Add 12 µl of Qiagen Buffer EB to the beads. Mix until homogenous by vortexing for 1 to 2 minutes at 2000 rpm. Spin the tubes down to pellet the beads and then place the tubes on the magnetic rack until the supernatant clears. Collect 10 µl of the eluted library.
16. Quantify 1 µl of sample library using a Qubit™ dsDNA BR Assay Kit and the Qubit 3.0 Fluorometer
17. Determine the size distribution of the library by running 1 µl (0.5-50 ng/µl) of sample library on a 2100 Bioanalyzer using an Agilent DNA 12000 Kit.

### Anneal and Bind SMRTbell Libraries

The DNA/Polymerase Binding and Magbead kits are required for this step. Use the Pacbio P6C4 Binding Calculator, version 2.3.1.1, to determine the conditions for annealing the sequencing primer and binding polymerase to SMRTbell libraries. See the Binding Calculator parameters below.

| **Sample Name** | **P1** | **P3** | **P5** | **P51** |
|---|---|---|---|---|
| Sample Volume to Use | 3 µl | 3 µl | 3 µl | 3 µl |
| # of SMRTCells | | | | |
| Concentration | 23.9 ng/uL | 15.7 ng/µl | 39.4 ng/µl | 26.9 ng/µl |
| Insert size | 6300 bp | 6300 bp | 6300 bp | 6300 bp |
| Size Selection | No | No | No | No |
| Protocol | MagBead OCPW | MagBead OCPW | MagBead OCPW | MagBead OCPW |
| Binding Kit | P6 | P6 | P6 | P6 |
| Preparation | Small | Small | Small | Small |
| Long-Term Storage | No | No | No | No |
| DNA Control | No | No | No | No |
| Complex Reuse | No | No | No | No |
| Standard Concentration | Yes | Yes | Yes | Yes |
| Concentration On Plate | Custom (0.0125 nM) | Custom (0.0125 nM) | Custom (0.0125 nM) | Custom (0.0125 nM) |
| Control To Template | Default (1.2%) | Default (1.2%) | Default (1.2%) | Default (1.2%) |
| Polymerase: Template Ratio | Default (10) | Default (10) | Default (10) | Default (10) |
| Primer:Template Ratio | Default (20) | Default (20) | Default (20) | Default (20) |

1. Dilute the Sequencing Primer by adding the reagents below into a new 200 µl PCR tube.
a. Sequencing Primer 1ul
b. Elution Buffer32.3 µl
2. Incubate at 80 °C for 2 minutes then hold at 4 °C. Conditioned sequencing primer may be stored at -20 °C and used for up to 30 days.
3. Annealing the diluted sequencing primer by adding the appropriate amount of reagents in the order listed below into 200 µl PCR tubes.

| **Sample Name** | **P1** | **P3** | **P5** | **P51** |
|---|---|---|---|---|
| Volume H20 | 13.6 µl | 7.9 µl | 24.3 µl | 15.7 µl |
| 10x Primer Buffer | 2.1 µl | 1.4 µl | 3.5 µl | 2.4 µl |
| Sample Volume | 3 µl | 3 µl | 3 µl | 3 µl |
| Diluted Sequencing Primer | 2.3 µl | 1.5 µl | 3.8 µl | 2.6 µl |
| Total Volume | 21 µl (at 0.8333 nM) | 13.8 µl (at 0.8333 nM) | 34.6 µl (at 0.8333 nM) | 23.6 µl (at 0.8333 nM) |

4. Incubate at 20 °C for 30 minutes.
5. Place the samples on ice.
6. Dilute SA-DNA Polymerase by adding the reagents below to a new 200 µl PCR tube.

| | | |
|---|---|---|
| a. | SA-DNA Polymerase P6 | 3 µl |
| b. | Binding Buffer v2 | 27 µl |
| c. | Total Volume (50 nM final) | 30 µl |

7. Add the following components to the primer-annealed libraries and mix by flicking the tubes. Spin down to collect the sample.

| **Sample Name** | **P1** | **P3** | **P5** | **P51** |
|---|---|---|---|---|
| dNTP | 3.5 µl | 2.3 µl | 5.8 µl | 3.9 µl |
| DTT | 3.5 µl | 2.3 µl | 5.8 µl | 3.9 µl |
| Binding Buffer v2 | 3.5 µl | 2.3 µl | 5.8 µl | 3.9 µl |
| Polymerase Dilution | 3.5 µl | 2.3 µl | 5.8 µl | 3.9 µl |
| Total Volume | 35 µl (at 0.5 nM) | 23 µl (at 0.5 nM) | 57.7 µl (at 0.5 nM) | 39.4 µl (at 0.5 nM) |

8. Close the PCR tubes and incubate at 30 °C for 30 minutes and then return to ice. The polymerase bound SMRTBell libraries can be stored at 4 °C for ∼7 days and should be sequenced within 10 days. Sequencing yield and quality begins to decline after 7 days.
9. Prepare the SMRTBell libraries for sequencing by mixing the following for each sample in new 200 µl PCR tubes.

| | | |
|---|---|---|
| a. | MagBead Binding Buffer | 8.8 µl |
| b. | Polymerase Bound Library | 0.22 µl |

10. Place the diluted libraries back on ice.
11. Mix the MagBeads by vortexing until they are homogenous.
12. For each sample, add 35 µl of Magbeads to an empty 1.5 ml LoBind tube. For more than one sample make a master mix and add 10 % to account for pipetting loss. In this case, we have four samples so 4.1 x 35 µl = 143.5 µl.
13. Centrifuge for a couple of seconds to collect the MagBeads and place them on a magnetic bead rack until the supernatant clears.
14. Discard the supernatant.
15. Add 143.5 µl of MagBead Wash Buffer to the beads and mix until homogenous by pipetting 10 times.
16. Centrifuge the beads for a couple of seconds to collect the beads and place back on the magnetic rack until the supernatant clears.
17. Discard the supernatant.
18. Add 143.5 ul of MagBead Binding Buffer to the beads and mix by pipetting 10 times.
19. Centrifuge the beads for a couple of seconds to the collect beads.
20. Aliquot 35 µl of beads to four separate 1.5 ml LoBind tubes and place on ice.
21. Place the beads on a magnetic bead rack until the supernatant clears and then discard the supernatant.
22. Add 9 µl of the diluted library, from step 10, to the corresponding bead tube. Cap the bead tubes and mix the beads until homogenous by flicking the tubes. Centrifuge for a couple of seconds to collect the beads and then place the beads back on ice.
23. Incubate the beads at 4 °C on a rotator for 30 minutes and then place back on ice.
24. While the beads are incubating, dilute the DNA Internal Control Complex in new 200 µl PCR tubes. Mix the following.
a. Control 1 Mix

| | | |
|---|---|---|
| i. | MagBead Binding Buffer | 99 µl |
| ii. | DNA Internal Control Complex | 1 µl |

b. Control 2 Mix

| | | |
|---|---|---|
| i. | MagBead Binding Buffer | 49 µl |
| ii. | Control 1 mix | 1 µl |

25. Centrifuge the beads from step 23 for a couple of seconds and then place on a magnetic bead rack until the supernatant clears. Discard the supernatant and place the beads back on ice.
26. Add 18 µl of MagBead Binding buffer to the beads and mix by flicking the tubes.
27. Place the beads on a magnetic bead rack until the supernatant clears and then discard the supernatant.
28. Add 18 µl of MagBead Wash buffer to the beads and mix by flicking the tubes.
29. Place the beads on a magnetic bead rack until the supernatant clears and then discard the supernatant.
30. Add 3 µl of the diluted Control 2 Mix and 42 µl of MagBead Binding buffer to the beads and mix by flicking the tubes. Centrifuge for a couple of seconds to collect the bead mixture and place on ice until use.
31. Place a new 96-well PCR plate on ice.
32. Mix the beads from step 30 until homogenous by pipetting and aliquot 45 µl of each of the four mixtures to separate wells in the same column (e.g., column 1, rows A-D) of the 96-well PCR plate that is on ice.

### Pacbio Loading and Data Collection Time

1. The Pacbio run was configured using Pacbio RS Remote software, version 2.3. The following settings were used:
   a. Collection Protocol = MagBead OneCellPerWell v1
   b. AcquisitionTime (Movie Time)=360
   c. InsertSize=5000
   d. StageHS=True
   e. SizeSelectionEnabled=False
   f. DNAControlComplex=2kb_control
   g. Use2ndLook=False
   h. NumberOfCollections=1
2. The PCR Plate containing the sequencing libraries, DNA Sequencing Reagents and SMRTCells were loaded into the Pacbio RS II. The libraries were sequenced for 360 minutes.

### Variant Detection and Quantification

The raw sequencing data and dengue virus reference genome (GenBank accession U87411; Dengue Virus type 2, Strain 16681) were imported into SMRTanalysis software version 2.3.0.140936.p5.167094 that consisted of Daemon 2.3.0.139497, SMRTpipe 2.3.0.139497, SMRT Portal 2.3.0.140893 and SMRT View 2.3.0.140836. The P1, P3, P5 and P51 reads were aligned to the dengue virus reference genome (GenBank accession U87411; Dengue Virus type 2, Strain 16681) using the RS_ReadsOfInsert_Mapping. 1 protocol of the Smrtanalysis software with the default mapping parameters and the following filtering parameters, Minimum Full Passes = 5, Minimum Predicted Accuracy = 90, Minimum Read Length of Insert = 5000, Maximum Read Length of Insert = 7000. The reads of insert fasta (fasta and fastq files can be used interchangeably) files generated by the RS_ReadsOfOnsert_Mapping. 1 protocol were aligned to the dengue virus (GenBank accession U87411; Dengue Virus type 2, Strain 16681) reference genome with BWA and variants at each attenuation site were identified from the alignment SAM files using the Sam2Tsv (http://lindenb.github.io/jvarkit/Sam2Tsv.html) and custom bash scripts. A custom perl script was used to quantify variants at the three attenuation sites of interest.

Four SMRTcells were sequenced, producing average about 8.7 kb reads of insert post filtration. The reads were aligned to the wildtype dengue virus reference genome (GenBank accession U87411) using BLASR and variants were called with QUIVER. The data are summarized in the Result section.

### Further RT-PCR Optimization to Produce a Single PCR Product (free of non-specific bands) using TDV2

The existing protocol from phase I did not produce a single and clean amplicon PCR product. Therefore, additional protocol optimizations and methods were attempted to produce a PCR product with a single amplicon. These optimizations included using a different combination of previously designed primers for reverse transcription and PCR, increasing the RT and touchdown PCR reaction temperatures. These various optimization steps are described below:

### Reverse Transcription and Touchdown PCR Optimization

To identify new combinations PCR primers for optimization, *in silico* or ePCR with previously designed primer sets was done, allowing for 30% primer binding site mismatch, with Unipro UGENE software. Based on the ePCR analysis that demonstrated low off target binding and our results with nested PCR, primers 5F and 3R were selected for optimization. Primer sequences are in Table 5.

### Two pairs of primers used for RT-PCR optimization in nested PCR reaction

**Table 5: Primer sequences and characteristics. Note that the 1R reverse primer is also the primer used for reverse transcription. The primers are in pairs.**

| **Name** | **Dir.** | **Sequence** | **Start** | **Stop** | **Tm** | **Product** |
|---|---|---|---|---|---|---|
| IF (SEQ ID | F | AAGACAGATTCTTTGAGGGAGCTA | 26 | 49 | 59 | 5368 |
| 1R (SEQ ID | R | GGTCTGTGAAATGGGCTTCG | 5393 | 5374 | 59 | 5368 |
| 5F (SEQ ID | F | GACAGATTCTTTGAGGGAGCTAAG | 28 | 51 | 59 | 5364 |
| 3R (SEQ ID | R | GTCTGTGAAATGGGCTTCGTC | 5392 | 5372 | 60 | 5364 |

First, reverse transcription was done using 6.5 µl of TDV2 viral RNA (∼10⁸ viral genome copies/µl) with primer 3R at two different reaction temperatures 50 °C and 55 °C. Then, touchdown PCR was done with primers 5F and 3R as follows: 95 °C for 30 sec, annealing at 68 °C for 20 sec with a 1 degree drop each cycle for 10 cycles (i.e., cycle 1 - 68 °C, cycle 2 - 67 °C, cycle 3- 65 °C...) and extension at 68 °C for 6 min. An additional 5 cycles were run at 95 °C for 30 sec, annealing at 53, 55.4, 57.2, 59.4, 61.9, 65.7, or 68 °C for 20 sec and extension at 68 °C for 6 min, giving a total of 15 cycles. PCR product was run in a 1% TAE agarose gel and the results show that a single PCR amplicon of expected size was produced.

### Introduction of Unique Molecular Identifiers

Unique molecular Identifiers (UMIs) are advantageous in that they can correct for artifacts produced by library preparation, such as PCR duplication and preferential amplification of certain variants. Also, UMIs have been shown to improve detection of rare variants by correcting for sequencing errors. The concept of UMI is that each RNA or DNA molecule is attached with a molecular tag or barcode. Amplification products showing same UMIs are duplication of same DNA molecule. Sequence reads having different UMIs are the original molecules. UMIs have the potential to increase the accuracy of detection of mutations at 1% frequency or lower by removing false positives. The target quantification can be better achieved by counting the number of UMIs in the reads rather than total reads, since total reads are skewed due to non-uniform amplification. After primers and RT-PCR conditions were optimized, the next step was to incorporate UMIs into the 3R reverse transcription primer and thus, into each viral molecule to improve the quantification of the observed variants.

A fully random 16-nucleotide UMI and a 5' PCR Primer IIA binding site were added to the 5' end of the 3R primer. Primer sequences and characteristics are shown in Table 6.

### Primers and oligonucleotide sequences used during incorporation of UMIs

**Table 6: Primer and oligonucleotide sequences and characteristics. 5F and UMI 3R were used to introduce UMI using the previously established successful amplification using 5F and 3R. SMARTER IIA and 5' PCR Primer IIA were used with UMI 3R when the template switching protocol was attempted.**

| **Name** | **Sequence** | **Start** | **Stop** | **Tm** | **Product** |
|---|---|---|---|---|---|
| 5F | GACAGATTCTTTGAGGGAGCTAAG | 28 | 51 | 59 | 5405 |
| 5'PCR Primer IIA | AAGCAGTGGTATCAACGCAGAGTAC | 28 | 51 | 59 | 5405 |
| SMARTER IIA | AAGCAGTGGTATCAACGCAGAGTAC XXXXX | | | | 5462 |
| UMI 3R | AAGCAGTGGTATCAACGCAGAGTAC NNNNNNNNNNNNNNNNGTCTGTGAA ATGGGCTTCGTC | | | | |

| | | | | | |
|---|---|---|---|---|---|
| X=undisclosed nucleotide based on proprietary sequence from Clontech Laboratories, Inc. | | | | | |

**Table 7: Primers and SEQ ID numbering**

| **Name** | **Direction** |
|---|---|
| 5F (SEQ ID NO:9) | F |
| 5' PCR Primer IIA (SEQ ID NO:21) | Both |
| SMARTER IIA | 5' RT Adapter |
| UMI 3R (SEQ ID NO:22) | 3' RT Adapter containing UMI |

Next, RT-PCR was performed with the optimized conditions using 6.5 µl of TDV2 viral RNA (∼10⁸ viral genome copies/µl). The PCR product was run in a 1% TAE agarose gel. Subsequently, large scale PCR was performed. A 0.45X Ampure XP bead purification was used to concentrate the PCR product and remove excess primers. Large scale PCR and Ampure XP bead purification gave strong amplification of a single band at the expected size.

### Pacbio Sequencing and Variant Analysis

The material from optimized RT-PCR reaction was used to construct a PacBio sequencing library using Pacbio's amplicon library prep protocol as described above.

### Quantification of TDV2 genomes Using Read Based and UMI Based Methods

Variants were not called with QUIVER as they were in Phase I. The reason is that typical variant calling algorithms do not maintain individual read ID information since variants are called based on read consensus and coverage at variant sites. Therefore, individual read information is not needed. However, to utilize UMI based quantification methods, multiple reads containing the same UMI and sequence are collapsed into a single instance of that read. Consequently, it is necessary to maintain individual read ID information when using UMI based quantification methods and as such, typical variant callers do not facilitate analysis with UMIs. Therefore, an alternative variant identification workflow was developed to facilitate the use of UMIs. Briefly, this workflow consisted of trimming off the SMARTER UMI 3R adapter sequence, extracting the UMI sequence and appending it to read ID of the TDV2 read of insert fastq file, aligning the trimmed fastq file to the Dengue virus reference genome (GenBank accession U87411; Dengue Virus type 2, Strain 16681) using BWA-mem and counting the revertants at each attenuation site.

### Apply Optimized Library Prep and Variant Detection Protocol to TDV2 Mono-Revertants

### Large Scale PCR to Incorporate UMIs into Mono-Revertants

Large scale PCR was performed as described above in the "Introduce Unique Molecular Identifiers into Each Viral Molecule" section. The PCR products were run in a 1% TAE agarose gel. The results show a single amplicon of expected size for each revertant.

### Pacbio Sequencing and Variant Analysis

The material was used to construct Pacbio sequencing libraries using Pacbio's amplicon library prep protocol as outlined above. One SMRTcell was sequenced per revertant.

Next, variant analysis was done as described in the "Quantify TDV2 Variants Using Read Based And UMI Based Methods" section.

### APPLY OPTIMIZED LIBRARY PREP AND VARIANT DETECTION PROTOCOL TO FOUR COMPLEX REVERTANT MIXTURES

### Preparation of complex revertant mixtures

Four complex revertant mixture pools were prepared based on the genome copies in each individual revertant as determined by qRT-PCR and transported to the analytical group without disclosing any information on the composition. Details of the pools are described in the result section.

### Large Scale PCR to Incorporate UMIs into Complex Revertant Mixtures

RT was performed on four complex revertant mixtures, Complex Mix 1-4, as described above in the "Introduce Unique Molecular Identifiers into Each Viral Molecule" section using 6.5 µl of viral RNA (∼ 2.8 x 10⁷ viral genome copies/µl) and the SMARTER UMI 3R primer. Next, large scale touchdown PCR was performed as described above and PCR products were run in a 1% TAE agarose gel. The results show a single amplicon of expected size for Complex Mix 1-4.

### Pacbio Sequencing and Variant Analysis

The material was used to construct PacBio sequencing libraries using Pacbio's amplicon library prep protocol as described above. One SMRTcell was sequenced per complex revertant mixture.

Next, variant analysis was done as described in the "Quantify TDV2 Variants Using Read Based and UMI Based Methods" section.

### RESULTS

### Optimization of Pacbio Sequencing for TDV2 Analysis

### RT-PCR OPTIMIZATION

In touchdown PCR reactions, there was a band present in all lanes in the expected size range, plus several non-specific amplification products.
Two best performing primer pairs (1 and 4) optimized for number of cycles in nested PCR gave strong amplification of an expected size band. Primer pair 1 had fewer non-specific products than pair 4 and was taken forward to annealing temperature optimization. The optimum of 11 cycles was chosen and a 0.45X Ampure XP bead purification was used to remove non-specific amplification products less than 3 kilobases.

### PacBio Sequencing and Variant Analysis

The material was used to construct a PacBio sequencing library as described above. One SMRTcell was sequenced, yielding 366 Mbp of data and 12,467 reads of insert. The Mean number of passes around each read of insert was 6.15 giving a mean read quality of 0.9828. The mean read of insert length was 5321 nucleotides.
*Note: The mean number of passes is the mean number of times a polymerase read the same molecule. Derived by the PacBio analytics software, PacBio sequencing templates are single stranded circles. The polymerase keeps going around until it falls off, becomes inactivated by the laser, or the run finishes.*

The alignment of reads to the dengue virus reference genome (GenBank accession U87411; Dengue Virus type 2, Strain 16681) initially identified eleven variants. The mapped reads of insert length are shown in Fig. 4.

The first three of these were poorly supported by low coverage were discarded from the analysis. The remaining variants were well supported by the percentage of reads calling each one. The data are summarized in Table 8.

**Table 8: Summary of the observed variants with the numbers of supporting reads.**

| **Position** | **Variant** | Reads total | Reads calling | Percentage |
|---|---|---|---|---|
| 57 | C > T | 11,917 | 11,846 | 99.4 |
| 524 | A > T | 11,121 | 11,080 | 99.6 |
| 592 | A > G | 12,236 | 12,212 | 99.8 |
| 900 | T > C | 12,403 | 11,853 | 95.6 |
| 2,055 | C > T | 11,339 | 11,297 | 99.6 |
| 2,579 | G > A | 11,710 | 11,461 | 97.9 |
| 4,018 | C > T | 11,849 | 11,812 | 99.7 |
| 5,270 | A > T | 11,983 | 11,882 | 99.1 |

The combinations of the three attenuation variants at positions, 57, 2579 and 5270 were examined and are summarized in Table 9.

**Table 9: Haplotype analysis of the three attenuation loci.**

| **Count** | **Percent** | **57** | **2579** | **5270** |
|---|---|---|---|---|
| 10472 | 96.703 | T | A | T |
| 28 | 0.259 | C | A | T |
| 15 | 0.139 | A | A | T |
| 12 | 0.111 | G | A | T |
| 201 | 1.856 | T | G | T |
| 5 | 0.046 | T | C | T |
| 48 | 0.443 | T | a | A |
| 21 | 0.194 | T | A | C |
| 17 | 0.157 | T | A | G |
| 2 | 0.018 | C | G | T |
| 1 | 0.009 | A | G | T |
| 4 | 0.037 | T | G | A |
| 1 | 0.009 | T | G | G |
| 1 | 0.009 | C | A | C |
| 1 | 0.009 | A | A | C |
| 0 | 0 | C | G | A |

The read counts are shown in the first column. The data are arranged such that observed variants are in order of positions 57, 2579 and 5270. Although PCR limits the ability to infer quantitative information about the input RNA, the TDV2 haplotype TAT is by far the most abundant. No reads having the reference haplotype CGA were found. The remainder have either one or two variants with respect to the TDV2 haplotype.

### IDENTIFICATION OF MONO REVERTANTS FROM BLINDED SAMPLES

### SANGER SEQUENCING TO IDENTIFY MONO REVERTANTS

Following conditions were selected as final optimized conditions:
RT at 55°C for 30 minutes
Annealing Temp.: 60°C
Number of PCR cycles: 30

Gel extraction kit from Qiagen was used to purify all PCR products of all TDV2 revertants. Sanger sequencing analysis confirmed the identity of TDV2 revertants (Table 10).

**Table 10: Summary of Sanger Sequencing Results**

| **Revertants** | **Reversion Site** | **Confirmed (Yes/No)** |
|---|---|---|
| P1 | A----NS1-2579----G | Yes |
| P3 | T----NS3-5270----A | Yes |
| P5 | T----5'NC-57----C | Yes |
| P51 | T----5'NC-57-----C A----NS1-2579---G | Yes |
| TDV2 | None - No Reversion | Yes |

### PAC BIO SEQUENCING TO IDENTIFY MONO REVERTANTS

### ANALYSIS OF MONO REVERTANTS

30 cycles were determined to be optimal because the PCR product began to smear up the gel at 35-40 cycles, which indicated PCR chimera.

### Pacbio Sequencing and Variant Analysis

The Pacbio sequencing statistics are shown in Table 11. The P1, P3, P5 and P51 reads were aligned to the dengue virus reference genome (GenBank accession U87411; Dengue Virus type 2, Strain 16681) using BLASR. The numbers of mapped reads are shown in Table 11. Variants were called with QUIVER. Haplotypes T:57-G:2579-T:5270, T:57-A:2579-A:5270, C:57-A:2579-T:5270 and C:57-G:2579-T:5270 were the most abundant haplotypes, making up > 99% of the population, in P1, P3, P5 and P51, respectively (Table **12**).

**Table 11: Pacbio sequencing and mapping statistics for mono-revertants, P1, P3, P5 and P51.**

| **Sample** | **Yield (Mbps)** | **Reads of Insert** | **Mean number of Passes** | **Mean Read Quality** | **Mean Read of Insert Length (bps)** | **Mapped Reads** |
|---|---|---|---|---|---|---|
| P1 | 49 | 9232 | 8.89 | 0.9955 | 5343 | 9240 |
| P3 | 29 | 5590 | 8.97 | 0.9955 | 5341 | 5596 |
| P5 | 59.7 | 11182 | 8.38 | 0.9954 | 5344 | 11199 |
| P51 | 47.9 | 8965 | 8.65 | 0.9957 | 5347 | 8975 |

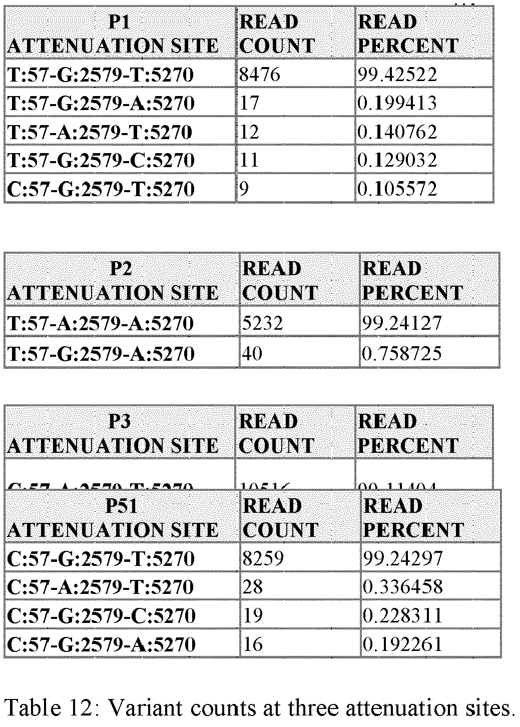

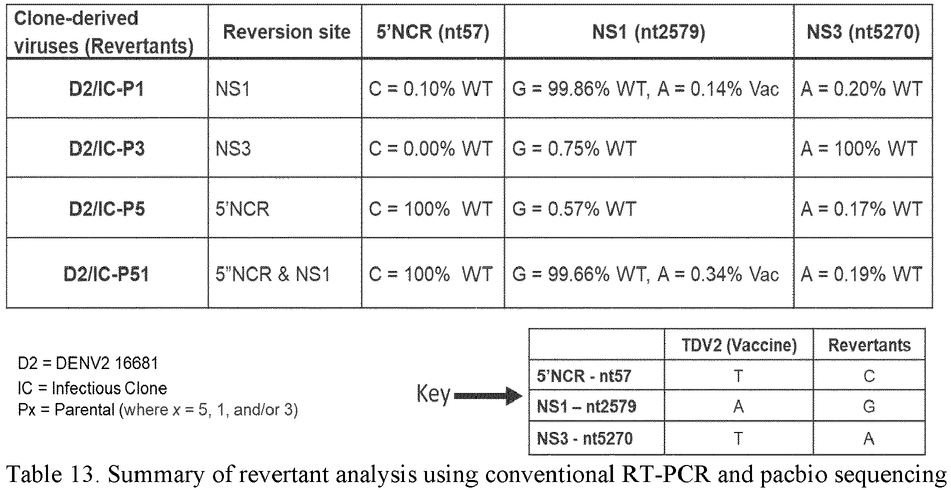

### Further RT-PCR Optimization to Produce a Single PCR Product (Using TDV2 viral RNA)

### Reverse Transcription and Touchdown PCR Optimization

Further optimization of RT and touchdown PCR reaction produced a single PCR amplicon of expected size. The results show that a single PCR amplicon of expected size was produced. Based on these results, 55 °C was determined to be the optimal RT temperature since there was less banding and smearing below 5 kbps. 57 °C was determined to be the optimal annealing temperature for the last five cycles of the PCR since the amplicon abundance begins to decline above 57.2 °C.

### Introduction of Unique Molecular Identifiers

The UMI sequences were added to gene specific reverse 3R primer. After adding UMIs, the RT-PCR was performed with the optimized conditions using 6.5 µl of TDV2 viral RNA (∼10⁸ viral genome copies/µl). The PCR product was run in a 1% TAE agarose gel. Subsequently, large scale PCR was performed. A 0.45X Ampure XP bead purification was used to concentrate the PCR product and remove excess primers. Large scale PCR and Ampure XP bead purification gave strong amplification of a single band.

### Pacbio Sequencing and Variant Analysis

One SMRTcell was sequenced and yielded 42.9 Mbps of data and 8038 reads of insert. The mean number of passes around each read of insert was 4.99 giving a mean read quality of 0.9838. The mean read of insert length was 5346 nucleotides.

### Quantify TDV2 genomes Using Read Based and UMI Based Methods

The variant counts using reads and UMIs are shown in Table 14. The results indicate that the T:57-A:2579-T:5270 TDV2 haplotype is by far the most common, while the wild-type haplotype (C:57-G:2579-A:5270) was not observed. However, some revertant haplotypes were present, including P1 (T:57-G:2579-T:5270), P3 (T:57-A:2579-A:5270) and P5 (C:57-A:2579-T:5270).

**Table 14: TDV2 variant counts using reads or UMIs at three attenuation sites.**

| **ATTENUATION SITE** | **READ COUNT** | **READ PERCENT** | **UMI COUNT** | **UMI PERCENT** |
|---|---|---|---|---|
| T:57-A:2579-T:5270 | 7041 | 96.17538588 | 6698 | 96.15274189 |
| T:57-G:2579-T:5270 | 184 | 2.513317853 | 177 | 2.540913006 |
| T:57-A:2579-G:5270 | 33 | 0.450758093 | 31 | 0.445018662 |
| G:57-A:2579-T:5270 | 18 | 0.245868051 | 17 | 0.244042492 |
| C:57-A:2579-T:5270 | 14 | 0.191230706 | 13 | 0.186620729 |
| A:57-A:2579-T:5270 | 9 | 0.122934025 | 8 | 0.114843526 |
| T:57-A:2579-C:5270 | 8 | 0.109274689 | 8 | 0.114843526 |
| T:57-C:2579-T:5270 | 6 | 0.081956017 | 6 | 0.086132644 |
| T:57-A:2579-A:5270 | 3 | 0.040978008 | 3 | 0.043066322 |

### Apply Optimized Library Prep and Variant Detection Protocol to TDV2 Mono-Revertants Large Scale PCR to Incorporate UMIs into Mono-Revertants

At this stage, the RT-PCR and library prep protocols were optimized. Therefore, we skipped the PCR optimization steps and proceeded directly to large scale PCR as described above in the "Introduce Unique Molecular Identifiers into Each Viral Molecule" section. The PCR products were run in a 1% TAE agarose gel. The results show a single amplicon of expected size for each revertant.

### Pacbio Sequencing and Variant Analysis

One SMRTcell was sequenced per revertant. Next, variant analysis was done as described in the "Quantify TDV2 Variants Using Read Based and UMI Based Methods" section. The results are shown in the following tables of Table 15 (1 of 4 to 4 of 4).

### Table 15: Variant Counts Tables using reads or UMIs at three attenuation loci for TDV2 mono revertants (P1, P3, P5 and P51)

**Table 15 (1 of 4): P1 variant counts using reads or UMIs at three attenuation sites.**

| **ATTENUATION SITE** | **READ COUNT** | **READ PERCENT** | **UMI COUNT** | **UMI PERCENT** |
|---|---|---|---|---|
| **T:57-G:2579-T:5270** | 11993 | 99.38675727 | 11121 | 99.39226026 |
| **C:57-G:2579-T:5270** | 30 | 0.248611917 | 29 | 0.259183126 |
| **T:57-G:2579-C:5270** | 13 | 0.107731831 | 13 | 0.116185539 |
| **T:57-A:2579-T:5270** | 12 | 0.099444767 | 11 | 0.098310841 |
| **T:57-G:2579-G:5270** | 7 | 0.058009447 | 7 | 0.062561444 |
| **G:57-G:2579-T:5270** | 7 | 0.058009447 | 5 | 0.044686746 |
| **T:57-T:2579-T:5270** | 2 | 0.016574128 | 2 | 0.017874698 |
| **T:57-G:2579-A:5270** | 2 | 0.016574128 | 2 | 0.017874698 |
| **A:57-G:2579-T:5270** | 1 | 0.008287064 | -1 | -0.008937349 |

**Table 15 (2 of 4): P3 variant counts using reads or UMIs at three attenuation sites.**

| **ATTENUATION SITE** | **READ COUNT** | **READ PERCENT** | **UMI COUNT** | **UMI PERCENT** |
|---|---|---|---|---|
| **T:57-A:2579-A:5270** | 7924 | 98.98813242 | 7542 | 98.97637795 |
| **T:57-G:2579-A:5270** | 52 | 0.649594004 | 49 | 0.643044619 |
| **C:57-A:2579-A:5270** | 11 | 0.137414116 | 11 | 0.144356955 |
| **T:57-A:2579-G:5270** | 4 | 0.04996877 | 4 | 0.052493438 |
| **T:57-T:2579-A:5270** | 4 | 0.04996877 | 4 | 0.052493438 |
| **T:57-C:2579-A:5270** | 3 | 0.037476577 | 3 | 0.039370079 |
| **T:57-A:2579-C:5270** | 2 | 0.024984385 | 2 | 0.026246719 |
| **T:57-A:2579-T:5270** | 2 | 0.024984385 | 2 | 0.026246719 |
| **A:57-G:2579-A:5270** | 1 | 0.012492192 | 1 | 0.01312336 |
| **G:57-A:2579-A:5270** | 1 | 0.012492192 | 1 | 0.01312336 |
| **T:57-G:2579-C:5270** | 1 | 0.012492192 | 1 | 0.01312336 |

**Table 15 (3 of 4): P5 variant counts using reads or UMIs at three attenuation sites.**

| **ATTENUATION SITE** | **READ COUNT** | **READ PERCENT** | **UMI COUNT** | **UMI PERCENT** |
|---|---|---|---|---|
| **C:57-A:2579-T:5270** | 9759 | 98.58571573 | 9177 | 98.6349957 |
| **C:57-G:2579-T:5270** | 79 | 0.79806041 | 72 | 0.773860705 |
| **C:57-A:2579-C:5270** | 33 | 0.333367007 | 29 | 0.311693895 |
| **C:57-A:2579-G:5270** | 10 | 0.101020305 | 9 | 0.096732588 |
| **T:57-A:2579-T:5270** | 7 | 0.070714214 | 6 | 0.064488392 |
| **A:57-A:2579-T:5270** | 3 | 0.030306092 | 3 | 0.032244196 |
| **C:57-A:2579-A:5270** | 3 | 0.030306092 | 3 | 0.032244196 |
| **C:57-C:2579-T:5270** | 2 | 0.020204061 | 2 | 0.021496131 |
| **C:57-T:2579-T:5270** | 1 | 0.010102031 | 1 | 0.010748065 |
| **G:57-A:2579-T:5270** | 1 | 0.010102031 | 1 | 0.010748065 |
| **G:57-G:2579-T:5270** | 1 | 0.010102031 | 1 | 0.010748065 |

**Table 15 (4 of 4): P51 variant counts using reads or UMIs at three attenuation sites.**

| **ATTENUATION SITE** | **READ COUNT** | **READ PERCENT** | **UMI COUNT** | **UMI PERCENT** |
|---|---|---|---|---|
| **C:57-G:2579-T:5270** | 11628 | 99.61449499 | 10810 | 99.60379619 |
| **C:57-A:2579-T:5270** | 16 | 0.137068449 | 14 | 0.128996591 |
| **C:57-G:2579-C:5270** | 15 | 0.128501671 | 15 | 0.138210633 |
| **C:57-G:2579-G:5270** | 6 | 0.051400668 | 6 | 0.055284253 |
| **C:57-G:2579-A:5270** | 3 | 0.025700334 | 3 | 0.027642127 |
| **T:57-G:2579-T:5270** | 3 | 0.025700334 | 3 | 0.027642127 |
| **A:57-G:2579-T:5270** | 1 | 0.008566778 | 1 | 0.009214042 |
| **C:57-C:2579-T:5270** | 1 | 0.008566778 | 1 | 0.009214042 |

The Pacbio sequencing statistics are shown in Table 16. The P1, P3, P5 and P51 reads were aligned to the dengue virus reference genome (GenBank accession U87411; Dengue Virus type 2, Strain 16681) using BLASR. The numbers of mapped reads are shown in Table 16.

Similar to the revertant analysis using the BLASR and Quiver workflow, haplotypes T:57-G:2579-T:5270, T:57-A:2579-A:5270, C:57-A:2579-T:5270 and C:57-G:2579-T:5270 were the most abundant in P1, P3, P5 and P51, respectively. But in contrast to the BLASR and Quiver workflow, which identified 2-5 haplotypes per revertant, the more recent **UMI workflow identified 8-11 haplotypes per revertant** (see Table 15).

**Table 16: Pacbio sequencing statistics for mono-revertants P1, P3, P5 and P51.**

| **Sample** | **Yield (Mbps)** | **Reads of Insert** | **Mean number of Passes** | **Mean Read Quality** | **Mean Read Of Insert Length (bps)** |
|---|---|---|---|---|---|
| P1 | 81.9 | 1754 | 10.15 | 0.9935 | 4669 |
| P3 | 52 | 11134 | 10.56 | 0.9948 | 4674 |
| P5 | 66 | 14072 | 9.24 | 0.9924 | 4699 |
| P51 | 65.6 | 12199 | 8.67 | 0.9955 | 5378 |

Analysis of the results showed expected changes in nucleotides at three main attenuation loci after RT-PCR and sequencing of these revertants. A total read of 8.7 K post filtration (size 5.4Kb) using **6500 copies of viral RNA** in a 30 cycle PCR reaction has been obtained.

### Quantitation of Percentage of revertants in a complex mixture

### Preparation of complex mixtures:

qRT-PCR was conducted on individual revertants and 4 separate mixes were prepared based on the genome copies as given in the following chart (Table 17).

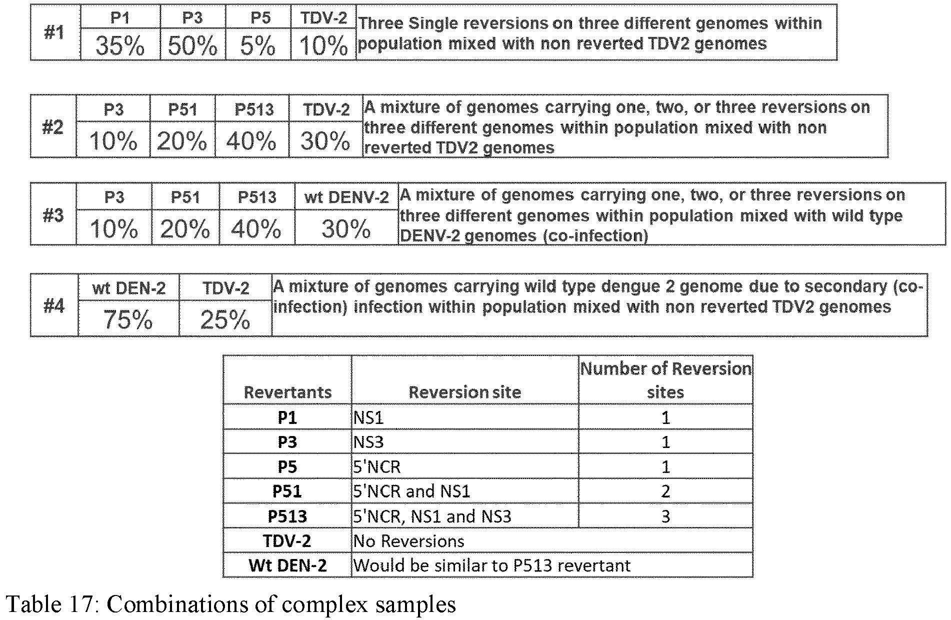

### Large Scale PCR to Incorporate UMIs into Complex Revertant Mixtures

PCR products were run in a 1% TAE agarose gel. The results show a single amplicon of expected size for Complex Mix 1-4. Large scale RT-PCR to incorporate UMI into complex revertant mixtures, complex mix 1-4. RT was done using the SMARTER IIA Oligonucleotide, followed by touchdown PCR with the 5F forward primer and the 5' PCR Primer IIA reverse primer.

### Pacbio Sequencing and Variant Analysis

One SMRTcell was sequenced per complex revertant mixture. The Pacbio sequencing statistics are shown in Table 18.

**Table 18: Pacbio sequencing stats for four complex revertant pools.**

| **Sample** | **Yield (Mbps)** | **Reads of Insert** | **Mean number of Passes** | **Mean Read Quality** | **Mean Read Of Insert Length (bps)** |
|---|---|---|---|---|---|
| P#1 | 52.3 | 9737 | 8.57 | 0.9951 | 5375 |
| P#2 | 44.5 | 8959 | 9.5 | 0.9961 | 4967 |
| P#3 | 49.7 | 9254 | 9.12 | 0.996 | 5380 |
| P#4 | 44.1 | 8195 | 8.95 | 0.9967 | 5388 |

### Table 19: Variant Counts Tables using reads or UMIs at three attenuation loci for complex mixtures of TDV2 mono revertants

**Table 19 (1 of 4): Complex Mix 1 (P#1) variant counts using reads or UMIs at three attenuation sites. The estimated % of haplotypes in the mix were 23.27 (P1), 57.76 (P3), 6.89 (P5) and 11.20 (TDV2).**

| **P#1 ATTENUATION SITE** | **READ COUNT** | **READ PERCENT** | **UMI COUNT** | **UMI PERCENT** |
|---|---|---|---|---|
| **T:57-A:2579-A:5270** | 5261 | 57.78143877 | 4955 | 57.76404756 |
| **T:57-G:2579-T:5270** | 2117 | 23.25096101 | 1996 | 23.26882723 |
| **T:57-A:2579-T:5270** | 1025 | 11.2575508 | 961 | 11.20307764 |
| **C:57-A:2579-T:5270** | 625 | 6.864360242 | 591 | 6.889717883 |
| **T:57-G:2579-A:5270** | 43 | 0.472267985 | 42 | 0.489624621 |
| **C:57-G:2579-T:5270** | 9 | 0.098846787 | 9 | 0.104919562 |
| **C:57-A:2579-A:5270** | 8 | 0.087863811 | 8 | 0.093261833 |
| **T:57-A:2579-G:5270** | 8 | 0.087863811 | 7 | 0.081604104 |
| **C:57-A:2579-C:5270** | 3 | 0.032948929 | 3 | 0.034973187 |
| **G:57-A:2579-A:5270** | 3 | 0.032948929 | 3 | 0.034973187 |
| **A:57-G:2579-T:5270** | 1 | 0.010982976 | 1 | 0.011657729 |
| **T:57-T:2579-A:5270** | 1 | 0.010982976 | 1 | 0.011657729 |
| **T:57-T:2579-T:5270** | 1 | 0.010982976 | 1 | 0.011657729 |

**Table 19 (2 of 4): Complex Mix 2 (P#2) variant counts using reads or UMIs at three attenuation sites. The estimated % of haplotypes in the mix were 10.84 (P3), 21.56 (P51), 37.16 (P513) and 29.59 (TDV-2).**

| **P#2 ATTENUATION SITE** | **READ COUNT** | **READ PERCENT** | **UMI COUNT** | **UMI PERCENT** |
|---|---|---|---|---|
| **C:57-G:2579-A:5270** | 2663 | 37.14086471 | 2558 | 37.16402731 |
| **T:57-A:2579-T:5270** | 2119 | 29.55369596 | 2037 | 29.59465349 |
| **C:57-G:2579-T:5270** | 1547 | 21.57601116 | 1484 | 21.56036612 |
| **T:57-A:2579-A:5270** | 780 | 10.87866109 | 746 | 10.83829725 |
| **T:57-G:2579-T:5270** | 17 | 0.237099024 | 16 | 0.232456778 |
| **T:57-G:2579-A:5270** | 11 | 0.153417015 | 11 | 0.159814035 |
| **C:57-A:2579-T:5270** | 9 | 0.125523013 | 9 | 0.130756937 |
| **C:57-A:2579-A:5270** | 8 | 0.111576011 | 6 | 0.087171292 |
| **T:57-A:2579-G:5270** | 5 | 0.069735007 | 5 | 0.072642743 |
| **C:57-G:2579-C:5270** | 2 | 0.027894003 | 2 | 0.029057097 |
| **C:57-T:2579-T:5270** | 2 | 0.027894003 | 2 | 0.029057097 |
| **A:57-A:2579-T:5270** | 1 | 0.013947001 | 1 | 0.014528549 |
| **C:57-G:2579-G:5270** | 1 | 0.013947001 | 1 | 0.014528549 |
| **G:57-A:2579-A:5270** | 1 | 0.013947001 | 1 | 0.014528549 |
| **G:57-A:2579-T:5270** | 1 | 0.013947001 | 1 | 0.014528549 |
| **T:57-A:2579-C:5270** | 1 | 0.013947001 | 1 | 0.014528549 |
| **T:57-C:2579-T:5270** | 1 | 0.013947001 | 1 | 0.014528549 |
| **T:57-T:2579-T:5270** | 1 | 0.013947001 | 1 | 0.014528549 |

**Table 19 (3 of 4): Complex Mix 3 (P#3) variant counts using reads or UMIs at three attenuation sites. The estimated % of haplotypes in the mix were 9.62 (P3), 25.12 (P51), 37.16 (P513) and 27.72 (wt DENV-2).**

| **P#3 ATTENUATION SITE** | **READ COUNT** | **READ PERCENT** | **UMI COUNT** | **UMI PERCENT** |
|---|---|---|---|---|
| **C:57-G:2579-A:5270** | 5740 | 64.75631769 | 5428 | 64.8894202 |
| **C:57-G:2579-T:5270** | 2237 | 25.23691336 | 2101 | 25.11655708 |
| **T:57-A:2579-A:5270** | 853 | 9.623194946 | 805 | 9.623430962 |
| **C:57-A:2579-A:5270** | 13 | 0.14666065 | 12 | 0.143454871 |
| **C:57-G:2579-C:5270** | 5 | 0.056407942 | 5 | 0.059772863 |
| **C:57-G:2579-G:5270** | 5 | 0.056407942 | 4 | 0.04781829 |
| **T:57-G:2579-A:5270** | 4 | 0.045126354 | 4 | 0.04781829 |
| **A:57-G:2579-A:5270** | 2 | 0.022563177 | 2 | 0.023909145 |
| **T:57-A:2579-T:5270** | 2 | 0.022563177 | 2 | 0.023909145 |
| **T:57-G:2579-T:5270** | 2 | 0.022563177 | 1 | 0.011954573 |
| **C:57-A:2579-T:5270** | 1 | 0.011281588 | 1 | 0.011954573 |

**Table 19 (4 of 4): Complex Mix 4 (P#4) variant counts using reads or UMIs at three attenuation sites. The estimated % of haplotypes in the mix were 65.57 (wt DENV-2) and 33.70 (TDV-2).**

| **P#4 ATTENUATION SITE** | **READ COUNT** | **READ PERCENT** | **UMI COUNT** | **UMI PERCENT** |
|---|---|---|---|---|
| **C:57-G:2579-A:5270** | 5159 | 65.64448403 | 4926 | 65.57507987 |
| **T:57-A:2579-T:5270** | 2645 | 33.65568138 | 2532 | 33.70607029 |
| **C:57-A:2579-T:5270** | 11 | 0.139966917 | 11 | 0.146432375 |
| **C:57-G:2579-T:5270** | 9 | 0.114518387 | 9 | 0.119808307 |
| **T:57-G:2579-A:5270** | 8 | 0.101794121 | 8 | 0.106496273 |
| **C:57-A:2579-A:5270** | 6 | 0.076345591 | 6 | 0.079872204 |
| **T:57-A:2579-A:5270** | 5 | 0.063621326 | 5 | 0.06656017 |
| **C:57-G:2579-G:5270** | 4 | 0.050897061 | 4 | 0.053248136 |
| **T:57-A:2579-C:5270** | 4 | 0.050897061 | 3 | 0.039936102 |
| **T:57-G:2579-T:5270** | 3 | 0.038172796 | 3 | 0.039936102 |
| **T:57-A:2579-G:5270** | 2 | 0.02544853 | 2 | 0.026624068 |
| **A:57-G:2579-A:5270** | 1 | 0.012724265 | 1 | 0.013312034 |
| **C:57-T:2579-A:5270** | 1 | 0.012724265 | 1 | 0.013312034 |
| **T:57-C:2579-T:5270** | 1 | 0.012724265 | 1 | 0.013312034 |

For Complex Mix 1, 13 haplotypes were identified with T:57-A:2579-A:5270 being the most abundant, followed by T:57-G:2579-T:5270, T:57-A:2579-T:5270 and C:57-A:2579-T:5270 (Table 19; 1 of 4); the remaining haplotypes made up a very small percentage of the population, ∼ 0.77 %. Complex Mix 2 contained the larget number of haplotypes, 18, with C:57-G:2579-A:5270 being the most abundant followed by T:57-A:2579-T:5270, C:57-G:2579-T:5270 and T:57-A:2579-A:5270; the remaining haplotypes made up ∼ 0.85% of the population (Table 19; 2 of 4). Complex Mix 3 contained the fewest numbers of haplotypes with 11. Haplotype C:57-G:2579-A:5270 was the most abundant, followed by C:57-G:2579-T:5270, and T:57-A:2579-A:5270, with the remaining haplotype making up ∼ 0.38 % of the population (Table 19; 3 of 4). Complex Mix 4 contained the second most haplotypes with 14. However, just two haplotypes, C:57-G:2579-A:5270 and T:57-A:2579-T:5270, made up over 99% of the population with the remaining haplotypes making up only ∼0.7 % of the population (Table 19; 4 of 4).

**Table 20. Read and UMI counting at attenuation sites for complex samples**

| **ATTENUATION SITES** | **READ_ COUNT** | **READ_PERCENT** | **UMI_ COUNT** | **UMI_PERCENT** |
|---|---|---|---|---|
| **P#1** | | | | |
| T:57-A:2579-A:5270 | 5261 | 57.78 | 4955 | 57.76 |
| T:57-G:2579-T:5270 | 2117 | 23.25 | 1996 | 23.26 |
| T:57-A:2579-T:5270 | 1025 | 11.25 | 961 | 11.20 |
| C:57-A:2579-T:5270 | 625 | 6.86 | 591 | 6.88 |

| **P#2** | | | | |
|---|---|---|---|---|
| C:57-G:2579-A;5270 | 2663 | 37.14 | 2558 | 37.16 |
| T:57-A;2579-T:5270 | 2119 | 29.55 | 2037 | 29.59 |
| C:57-G:2579-T:5270 | 1547 | 21.57 | 1484 | 21.56 |
| T:57-A:2579-A:5270 | 780 | 10.87 | 746 | 10.83 |

| **P#3** | | | | |
|---|---|---|---|---|
| C:57-G:2579-A:5270 | 5740 | 64.75 | 5428 | 64.88 |
| C:57-G:2579-T:5270 | 2237 | 25.23 | 2101 | 25.11 |
| T:57-A:2579-A:5270 | 853 | 9.62 | 805 | 9.62 |

| **P#4** | | | | |
|---|---|---|---|---|
| C:57-G:2579-A:5270 | 5159 | 65.64 | 4926 | 65.57 |
| T:57-A:2579-T:5270 | 2645 | 33.65 | 2532 | 33.70 |

| | | | | |
|---|---|---|---|---|
| Wild-Type: C:57-G:2579-A:5270 Vaccine: T:57-A:2579-T:5270 | | | | |

### Comparison of pacbio results with blinded complex samples

The percentage of each revertant in the complex mix determined by the pacbio sequencing was compared with the blinded mix prepared by Takeda. As can be seen from the Table 21, the quantity of each haplotype in the pools P#1, P#2, P#3 and P#4 was comparable to that of original amounts formulated by Takeda.

**Table 21: Quantitation of haplotypes in the complex pools by pacbio sequencing**

| **Pool #** | **Components of mix** | | | |
|---|---|---|---|---|
| **1** | **P1** | **P3** | **P5** | **TDV-2** |
| | 35 | 50 | 5 | 10 |
| | 23.27 | 57.76 | 6.89 | 11.20 |
| **2** | **P3** | **P51** | **P513** | **TDV-2** |
| | 10 | 20 | 40 | 30 |
| | 10.84 | 21.56 | 37.16 | 29.59 |
| **3** | **P3** | **P51** | **P513** | **Wt DENV-2** |
| | 10 | 20 | 40 | 30 |
| | 9.62 | 25.12 | 64.89 (37.16 + 27.72) | |
| **4** | **Wt DENV-2** | **TDV-2** | - | |
| | 75 | 25 | - | |
| | 65.57 | 33.70 | - | |

### IDENTITY BY RT-PCR AMPLIFICATION OF E GENE SEQUENCES

Reverse transcription polymerase chain reaction (RT-PCR) is a sensitive method for the detection of RNA, which involves two step reactions. RNA is first reverse transcribed to cDNA by reverse transcriptase. The cDNA is then used as template for the subsequent PCR step. Amplified DNA fragments are separated and visualized in a 2% agarose gel; method conditions are listed in Table 23. The identity of each monovalent drug substance is determined by a positive result for RT-PCR amplification of an E gene sequence specific to the appropriate dengue serotype, and a negative result for RT-PCR amplification of the E gene sequences specific to the other three dengue serotypes. Four primer sets were designed to differentiate the four serotypes of the dengue vaccine viruses. The serotype-specific primer sets were designed such that each primer has only one specific binding site within the genome of the corresponding dengue serotype and each primer set generates a DNA fragment of specific size. Viral RNA is isolated from the test sample using the QIAamp® cador® Pathogen Kit according to the manufacturer's instructions. RT-PCR on the isolated viral RNA is performed using a one-step RT-PCR kit (highQu GmbH). The amplicons for the four vaccine virus strains are 419, 390, 731 and 226 base pairs for TDV-1, TDV-2, TDV-3 and TDV-4, respectively, and are easily differentiated on a 2% agarose gel (Table 22).

**Table 22 Primer Sequences and Size of Amplicons for RT-PCR-based Identity Test**

| **Virus Strain** | | **Serotype-Specific E Gene Primer Sequences** | **Amplicon Size (in Basepairs)** |
|---|---|---|---|
| TDV-1 | Forward | 5'-CCGACTACGGAACCCTTACA-3' | 419 |
| | Reverse | 5'-GGTCTCAGCCACTTCTTTCTCTA-3' | |
| TDV-2 | Forward | 5'-GCCATGCACACAGCACTTAC-3' | 390 |
| | Reverse | 5'-CTTCAGTTGTCCCGGCTCTA-3' | |
| TDV-3 | Forward | 5'-AAGCCCACGCTGGACATAG-3' | 731 |
| | Reverse | 5'-AAGTGCCCCGCAAAGATACT-3' | |
| TDV-4 | Forward | 5'-GCCGGAGAGATGTGGTAGAC-3' | 226 |
| | Reverse | 5'-CTGGGAGTTATCGTGGCTGTA-3' | |

The above primer sequences have been assigned the following sequence identifiers.

| | | |
|---|---|---|
| TDV-1 | Forward | SEQ ID NO:24 |
| | Reverse | SEQ ID NO:25 |
| TDV-2 | Forward | SEQ ID NO:26 |
| | Reverse | SEQ ID NO:27 |
| TDV-3 | Forward | SEQ ID NO:28 |
| | Reverse | SEQ ID NO:29 |
| TDV-4 | Forward | SEQ ID NO:30 |
| | Reverse | SEQ ID NO:31 |

**Table 23: RT-PCR Condition**

| **Temperature** | **Time** | **Cycles** |
|---|---|---|
| 50 °C | 20 mm | 1 |
| 95 °C | 2 min | 1 |
| 95 °C | 30 sec | 35 |
| 66 °C | 30 sec | |
| 72 °C | 1 min | |
| 4 °C | 0 to infinity | 1 |

### RESIDUAL HOST CELL DNA BY qPCR

A qPCR based method is used for residual host cell DNA that detects and estimates Vero DNA.

This method utilizes oligo-magnetic bead capture to improve DNA detection and is available in a kit manufactured by Life Technologies Inc. The method was employed to ensure low levels of residual Vero host cell DNA can be detected in the bulk drug substance.
For DNA isolation and purification, the PrepSEQ® Residual DNA Sample Preparation Kit extracts host cell DNA from products produced in Vero cells. The kit uses chemical lysis and magnetic beads to efficiently extract genomic DNA from diverse sample types, including samples that contain high protein and low DNA concentration. Each test sample is extracted in triplicate and a single PCR reaction is performed for each extraction.

For quantitative PCR, the resDNASEQ® Quantitative DNA kit is used to quantify residual DNA from the Vero cell line which is used for production of bulk drug substance. The resDNASEQ® Quantitative DNA kit uses TaqMan® quantitative PCR to perform rapid, specific quantitation of low levels of residual host cell DNA in testing samples by interpolation of the standard curve provided in the kit.

There are 4 controls used for the DNA extraction and qPCR. Extraction Recovery Control (ERC) is drug substance sample spiked with 500 pg of Vero Control DNA. Negative Control (NC) is molecular biology grade water used as negative control for the whole procedure, from DNA extraction to qPCR. No Template Control (NTC) is molecular biology grade water as negative control in qPCR. Internal Positive Control (IPC) is provided in the qPCR kit as positive control for qPCR.

For the testing to be valid, the standard curve generated must fulfill the conditions of R2 more than 0.99, efficiency of 90% to 110%, and y-intercept between 24 and 29. The NTC and SE must have a mean of triplicate negative controls DNA quantity of less than 30 fg. The ERC must have percentage recovery mean of triplicate between 50% and 150%. The IPC must have a Ct value less than 40.00 in all test samples and controls. For the sample result to be valid, the repeatability of the test sample replicates must be less than or equal to 25% CV.
The Ct values of valid test samples from the qPCR are used to determine the result. The result is reported as 'Negative' if the Ct is undetermined. The result is reported as 'Positive' if the Ct values of at least 2 out of 3 replicates fall within the standard curve. The DNA concentration will be calculated by interpolating the Ct values from the standard curve.

## Claims

1. A method for detecting the presence of at least one revertant virus in a sample containing an attenuated virus comprising the steps:
a) purifying virus nucleic acids from said sample;
b) optionally, preparing double-stranded DNA templates from said virus nucleic acids;
c) amplifying said viral nucleic acids of a) or said double-stranded DNA templates of b) by polymerase chain reaction (PCR) such that PCR products having a length of at least 2 kb and comprising at least one attenuation locus of said attenuated virus are generated;
d) sequencing said PCR products by single-molecule real-time (SMRT) sequencing; and
e) comparing at least one nucleotide at said at least one attenuation locus in said PCR products with the corresponding nucleotide in the wild-type virus sequence, wherein the presence of the wild-type nucleotide at said at least one attenuation locus is indicative of the presence of a revertant virus in said sample.

2. The method of claim 1, wherein said virus is an RNA virus, preferably wherein the RNA virus is selected from dengue virus, poliovirus, rubella virus, measles virus, yellow fever virus, mumps virus and influenza virus.

3. The method of claim 1 or 2, wherein said PCR products have a length of at least 3 kb, preferably of at least 5 kb.

4. The method of any one of claims 1 to 3, wherein said PCR products comprise at least two attenuation loci, preferably at least three attenuation loci.

5. The method of any one of claims 1 to 4, wherein said attenuated virus is a live, attenuated dengue virus and said at least one attenuation locus is selected from positions 57, 2579 and/or 5270 of the nucleotide sequence of dengue virus type 2 according to SEQ ID NO: 23.

6. The method of any one of claims 1 to 5, wherein prior to step a) the live, attenuated virus is grown in Vero cells.

7. The method of any one of claims 1 to 6, wherein the virus nucleic acids are isolated virus RNA and step b) comprises a step of reverse transcription of the isolated virus RNA in the presence of a reverse transcriptase using a reverse primer.

8. The method of claim 7, wherein the reverse transcription is performed using as reverse primer an oligonucleotide comprising the nucleotide sequence of SEQ ID NO:6 (3R) at a temperature in the range from about 45°C to about 65° C, preferably at a temperature in the range from about 50°C to about 55°C.

9. The method of claim 1 to 8, wherein the PCR amplification reaction comprises the use of a first primer comprising the nucleotide sequence of SEQ ID NO:6 (3R) and a second primer comprising the nucleotide sequence of SEQ ID NO:9 (5F).

10. The method of any one of claims 1 to 9, wherein prior to step d) the method additionally comprises:
i) end repairing of the PCR products;
ii) optionally, purifying the end repaired PCR product; and
iii) connecting the two complementary strands of the PCR product prepared in step c) with a linking oligonucleotide, wherein the 3' end of one complementary strand is linked to the 5' end of the other complementary strand via the linking oligonucleotide in each double stranded nucleic acid fragment, whereby the linking oligonucleotide provides a single stranded portion of the resulting linked nucleic acid fragments.

11. A method for the quantitative analysis of a mixture of virus haplotypes in a virus sample comprising the steps of:
a) purifying virus nucleic acids from said sample;
b) preparing double-stranded DNA templates from said virus nucleic acids by using a primer comprising a unique molecular identifier (UMI) sequence such that said double-stranded DNA templates comprise the UMI sequence;
c) amplifying said double-stranded DNA templates by polymerase chain reaction (PCR) such that PCR products having a length of at least 2kb and comprising a unique molecular identifier (UMI) sequence are generated;
d) sequencing said PCR products by single-molecule real-time (SMRT) sequencing; and determining the relative amounts of said PCR products by analysing the UMI count and the Read count, thereby quantifying the virus haplotypes contained in the sample.

12. The method of claim 11, wherein any of the virus haplotypes is present in the virus sample in a quantity of less than 1 %, preferably less than 0.2 % based on the total amount of virus in the sample.

13. The method of claim 11 or 12, wherein the virus is an RNA virus, preferably wherein the RNA virus is selected from dengue virus, poliovirus, rubella virus, measles virus, yellow fever virus, mumps virus and influenza virus.

14. The method of any one of claims 11 to 13, wherein said virus is a live, attenuated dengue virus, preferably a dengue virus comprising at least one attenuation locus selected from positions 57, 2579 and/or 5270 of the nucleotide sequence of dengue virus type 2 according to SEQ ID NO: 23.

15. The method of any one of claims 11 to 14, wherein said PCR products have a length of at least 3 kb, preferably of at least 5 kb.

16. The method of any one of claims 11 to 15, wherein the virus nucleic acids are isolated virus RNA and step b) comprises a step of reverse transcription of the isolated virus RNA in the presence of a reverse transcriptase using a reverse primer additionally comprising at its 5'end a UMI sequence.

17. The method of claim 16, wherein the reverse transcription is performed using as reverse primer an oligonucleotide comprising the nucleotide sequence of SEQ ID NO:22 (UMI 3R) at a temperature in the range from about 45°C to about 65° C, preferably the oligonucleotide consists of the nucleotide sequence of SEQ ID NO:22 and the temperature is in the range from about 50°C to about 55°C.

18. The method of any one of claims 11 to 17, wherein the PCR amplification reaction comprises the use of a first primer comprising the nucleotide sequence of SEQ ID NO:21 (Primer IIA) and a second primer comprising the nucleotide sequence of SEQ ID NO: 9 (5F).

19. The method of claim 18, wherein after the amplification step c) and prior to the sequencing step d) the method additionally comprises:
i) end repairing of the PCR products;
ii) optionally, purifying the end repaired PCR product; and
iii) connecting the two complementary strands of the double stranded nucleic acid fragments prepared in step (b) with a linking oligonucleotide, wherein the 3' end of one complementary strand is linked to the 5' end of the other complementary strand via the linking oligonucleotide in each double stranded nucleic acid fragment, whereby the linking oligonucleotide provides a single stranded portion of the resulting linked nucleic acid fragments.

20. The method according to any one of claims 1 to 19, wherein said sample is a composition comprising an attenuated virus, or a sample from a patient.

21. The method of any one of claims 1 to 20, wherein prior to step d) the method comprises:
i) determining the sequence of the PCR products at at least one of the attenuation locus separately by Sanger sequencing;
ii) comparing at least one nucleotide at said at least one attenuation locus with the wild-type virus sequence; and
iii) if the wild-type sequence is determined at at least one attenuation locus, sequencing said PCR products by SMRT sequencing of step d) is carried out.

22. Use of the method according to any one of claims 1 to 10 in the quality control of vaccines containing live, attenuated virus.

23. Use of the method according to any one of claims 11 to 21 in the diagnosis of a virus infection in a patient sample.

24. A kit for detecting the presence of at least one revertant virus in a sample containing an attenuated dengue virus comprising a first primer selected from an oligonucleotide having the nucleotide sequence of SEQ ID NO: 1, SEQ ID NO: 3, SEQ ID NO: 5, SEQ ID NO: 7, SEQ ID NO:9 and SEQ ID NO:11, and comprising a second primer selected from an oligonucleotide having the nucleotide sequence of SEQ ID NO: 2, SEQ ID NO: 4, SEQ ID NO: 6, SEQ ID NO:8, SEQ ID NO: 10 and SEQ ID NO: 12, preferably the first primer comprises the nucleotide sequence of SEQ ID NO: 9 (5F) and the second primer comprises the nucleotide sequence of SEQ ID NO: 6 (3R).

25. A kit for the quantitative analysis of a mixture of virus haplotypes in a dengue virus sample comprising a first primer comprising the nucleotide sequence of SEQ ID NO: 9 (5F) and a second primer comprising the nucleotide sequence of SEQ ID NO:21 (Primer IIA).


SEQUENCE LISTING

<110>  Takeda Vaccines, Inc.

<120>  Qualitative and quantitative determination of single virus
       haplotypes in complex samples

<130>  T08285EP

<160>  31

<170>  PatentIn version 3.5

<210>  1
<211>  24
<212>  DNA
<213>  artificial

<220>
<223>  artificial

<400>  1
aagacagatt ctttgaggga gcta                                              24


<210>  2
<211>  20
<212>  DNA
<213>  artificial

<220>
<223>  artificial

<400>  2
ggtctgtgaa atgggcttcg                                                   20


<210>  3
<211>  23
<212>  DNA
<213>  artificial

<220>
<223>  artificial

<400>  3
gacagattct ttgagggagc taa                                               23


<210>  4
<211>  20
<212>  DNA
<213>  artificial

<220>
<223>  artificial

<400>  4
gggtctgtga aatgggcttc                                                   20


<210>  5
<211>  23

<212>  DNA
<213>  artificial

<220>
<223>  artificial

<400>  5
acagattctt tgagggagct aag                                               23


<210>  6
<211>  21
<212>  DNA
<213>  artificial

<220>
<223>  artificial

<400>  6
gtctgtgaaa tgggcttcgt c                                                 21


<210>  7
<211>  25
<212>  DNA
<213>  artificial

<220>
<223>  artificial

<400>  7
agacagattc tttgagggag ctaag                                             25


<210>  8
<211>  20
<212>  DNA
<213>  artificial

<220>
<223>  artificial

<400>  8
gtctgtgaaa tgggcttcgt                                                   20


<210>  9
<211>  24
<212>  DNA
<213>  artificial

<220>
<223>  artificial

<400>  9
gacagattct ttgagggagc taag                                              24


<210>  10
<211>  21
<212>  DNA
<213>  artificial

<220>
<223>  artificial

<400>  10
cttgctgggt ctgtgaaatg g                                                21


<210>  11
<211>  25
<212>  DNA
<213>  artificial

<220>
<223>  artificial

<400>  11
aagacagatt ctttgaggga gctaa                                            25


<210>  12
<211>  21
<212>  DNA
<213>  artificial

<220>
<223>  artificial

<400>  12
tgggtctgtg aaatgggctt c                                                21


<210>  13
<211>  24
<212>  DNA
<213>  artitificial

<400>  13
agttgttagt ctacgtggac cgac                                             24


<210>  14
<211>  30
<212>  DNA
<213>  artificial

<220>
<223>  artificial

<400>  14
gtgacactgt atttgggagt catggtgcag                                       30


<210>  15
<211>  25
<212>  DNA
<213>  artificial

<220>
<223>  artificial

<400>  15
ggtaatggtg ttgttacaag gagtg                                            25

<210>  16
<211>  24
<212>  DNA
<213>  artificial

<220>
<223>  artificial

<400>  16
aagcccttag aggacttcca ataa                                              24


<210>  17
<211>  24
<212>  DNA
<213>  artificial

<220>
<223>  artificial

<400>  17
tcagcatcct tccaatctct ttcc                                              24


<210>  18
<211>  24
<212>  DNA
<213>  artificial

<220>
<223>  artificial

<400>  18
ccgcagagat cgttttcctg cctg                                              24


<210>  19
<211>  25
<212>  DNA
<213>  artificial

<220>
<223>  artificial

<400>  19
ctaactggtg atagcagcct catgg                                             25


<210>  20
<211>  28
<212>  DNA
<213>  artificial

<220>
<223>  artificial

<400>  20
gaaatgggct tcgtccatga taatcagg                                          28


<210>  21
<211>  25

<212>  DNA
<213>  artificial

<220>
<223>  artificial

<400>  21
aagcagtggt atcaacgcag agtac                                              25


<210>  22
<211>  62
<212>  DNA
<213>  artificial

<220>
<223>  artificial


<220>
<221>  misc_feature
<222>  (26)..(41)
<223>  n is a, c, g, or t

<400>  22
aagcagtggt atcaacgcag agtacnnnnn nnnnnnnnnn ngtctgtgaa atgggcttcg        60

tc                                                                       62


<210>  23
<211>  10723
<212>  DNA
<213>  dengue serotype 2 (MVS)

<400>  23
agttgttagt ctacgtggac cgacaaagac agattctttg agggagctaa gctcaatgta        60

gttctaacag ttttttaatt agagagcaga tctctgatga ataaccaacg gaaaaaggcg       120

aaaaacacgc ctttcaatat gctgaaacgc gagagaaacc gcgtgtcgac tgtgcaacag       180

ctgacaaaga gattctcact tggaatgctg cagggacgag gaccattaaa actgttcatg       240

gccctggtgg cgttccttcg tttcctaaca atcccaccaa cagcagggat attgaagaga       300

tggggaacaa ttaaaaaatc aaaagctatt aatgttttga gagggttcag gaaagagatt       360

ggaaggatgc tgaacatctt gaataggaga cgcagatctg caggcatgat cattatgctg       420

attccaacag tgatggcgtt ccatttaacc acacgtaacg gagaaccaca catgatcgtc       480

agcagacaag agaaagggaa aagtcttctg tttaaaacag aggttggcgt gaacatgtgt       540

accctcatgg ccatggacct tggtgaattg tgtgaagaca caatcacgta cgagtgtccc       600

cttctcaggc agaatgagcc agaagacata gactgttggt gcaactctac gtccacgtgg       660

gtaacttatg ggacgtgtac caccatggga gaacatagaa gagaaaaaag atcagtggca       720

ctcgttccac atgtgggaat gggactggag acacgaactg aaacatggat gtcatcagaa       780

ggggcctgga aacatgtcca gagaattgaa acttggatct tgagacatcc aggcttcacc       840

atgatggcag caatcctggc atacaccata ggaacgacac atttccaaag agccctgatc 900

ttcatcttac tgacagctgt cactccttca atgacaatgc gttgcatagg aatgtcaaat 960

agagactttg tggaaggggt ttcaggagga agctgggttg acatagtctt agaacatgga 1020

agctgtgtga cgacgatggc aaaaaacaaa ccaacattgg attttgaact gataaaaaca 1080

gaagccaaac agcctgccac cctaaggaag tactgtatag aggcaaagct aaccaacaca 1140

acaacagaat ctcgctgccc aacacaaggg gaacccagcc taaatgaaga gcaggacaaa 1200

aggttcgtct gcaaacactc catggtagac agaggatggg gaaatggatg tggactattt 1260

ggaaagggag gcattgtgac ctgtgctatg ttcagatgca aaaagaacat ggaaggaaaa 1320

gttgtgcaac cagaaaactt ggaatacacc attgtgataa cacctcactc aggggaagag 1380

catgcagtcg gaaatgacac aggaaaacat ggcaaggaaa tcaaaataac accacagagt 1440

tccatcacag aagcagaatt gacaggttat ggcactgtca caatggagtg ctctccaaga 1500

acgggcctcg acttcaatga gatggtgttg ctgcagatgg aaaataaagc ttggctggtg 1560

cacaggcaat ggttcctaga cctgccgtta ccatggttgc ccggagcgga cacacaaggg 1620

tcaaattgga tacagaaaga gacattggtc actttcaaaa atccccatgc gaagaaacag 1680

gatgttgttg ttttaggatc ccaagaaggg gccatgcaca cagcacttac aggggccaca 1740

gaaatccaaa tgtcatcagg aaacttactc ttcacaggac atctcaagtg caggctgaga 1800

atggacaagc tacagctcaa aggaatgtca tactctatgt gcacaggaaa gtttaaagtt 1860

gtgaaggaaa tagcagaaac acaacatgga acaatagtta tcagagtgca atatgaaggg 1920

gacggctctc catgcaagat cccttttgag ataatggatt tggaaaaaag acatgtctta 1980

ggtcgcctga ttacagtcaa cccaattgtg acagaaaaag atagcccagt caacatagaa 2040

gcagaacctc catttggaga cagctacatc atcataggag tagagccggg acaactgaag 2100

ctcaactggt ttaagaaagg aagttctatc ggccaaatgt ttgagacaac aatgaggggg 2160

gcgaagagaa tggccatttt aggtgacaca gcctgggatt ttggatcctt gggaggagtg 2220

tttacatcta taggaaaggc tctccaccaa gtctttggag caatctatgg agctgccttc 2280

agtggggttt catggactat gaaaatcctc ataggagtca ttatcacatg gataggaatg 2340

aattcacgca gcacctcact gtctgtgaca ctagtattgg tgggaattgt gacactgtat 2400

ttgggagtca tggtgcaggc cgatagtggt tgcgttgtga gctggaaaaa caaagaactg 2460

aaatgtggca gtgggatttt catcacagac aacgtgcaca catggacaga acaatacaag 2520

ttccaaccag aatccccttc aaaactagct tcagctatcc agaaagccca tgaagaggac 2580

atttgtggaa tccgctcagt aacaagactg gagaatctga tgtggaaaca aataacacca 2640

gaattgaatc acattctatc agaaaatgag gtgaagttaa ctattatgac aggagacatc 2700

aaaggaatca tgcaggcagg aaaacgatct ctgcggcctc agcccactga gctgaagtat 2760

tcatggaaaa catggggcaa agcaaaaatg ctctctacag agtctcataa ccagaccttt 2820

ctcattgatg gccccgaaac agcagaatgc cccaacacaa atagagcttg gaattcgttg 2880

gaagttgaag actatggctt tggagtattc accaccaata tatggctaaa attgaaagaa 2940

aaacaggatg tattctgcga ctcaaaactc atgtcagcgg ccataaaaga caacagagcc 3000

gtccatgccg atatgggtta ttggatagaa agtgcactca atgacacatg gaagatagag 3060

aaagcctctt tcattgaagt taaaaactgc cactggccaa aatcacacac cctctggagc 3120

aatggagtgc tagaaagtga gatgataatt ccaaagaatc tcgctggacc agtgtctcaa 3180

cacaactata gaccaggcta ccatacacaa ataacaggac catggcatct aggtaagctt 3240

gagatggact ttgatttctg tgatggaaca acagtggtag tgactgagga ctgcggaaat 3300

agaggaccct ctttgagaac aaccactgcc tctggaaaac tcataacaga atggtgctgc 3360

cgatcttgca cattaccacc gctaagatac agaggtgagg atgggtgctg gtacgggatg 3420

gaaatcagac cattgaagga gaaagaagag aatttggtca actccttggt cacagctgga 3480

catgggcagg tcgacaactt ttcactagga gtcttgggaa tggcattgtt cctggaggaa 3540

atgcttagga cccgagtagg aacgaaacat gcaatactac tagttgcagt ttcttttgtg 3600

acattgatca cagggaacat gtcctttaga gacctgggaa gagtgatggt tatggtaggc 3660

gccactatga cggatgacat aggtatgggc gtgacttatc ttgccctact agcagccttc 3720

aaagtcagac caacttttgc agctggacta ctcttgagaa agctgacctc caaggaattg 3780

atgatgacta ctataggaat tgtactcctc tcccagagca ccataccaga gaccattctt 3840

gagttgactg atgcgttagc cttaggcatg atggtcctca aaatggtgag aaatatggaa 3900

aagtatcaat tggcagtgac tatcatggct atcttgtgcg tcccaaacgc agtgatatta 3960

caaaacgcat ggaaagtgag ttgcacaata ttggcagtgg tgtccgtttc cccactgttc 4020

ttaacatcct cacagcaaaa aacagattgg ataccattag cattgacgat caaaggtctc 4080

aatccaacag ctatttttct aacaaccctc tcaagaacca gcaagaaaag gagctggcca 4140

ttaaatgagg ctatcatggc agtcgggatg gtgagcattt tagccagttc tctcctaaaa 4200

aatgatattc ccatgacagg accattagtg gctggagggc tcctcactgt gtgctacgtg 4260

ctcactggac gatcggccga tttggaactg gagagagcag ccgatgtcaa atgggaagac 4320

caggcagaga tatcaggaag cagtccaatc ctgtcaataa caatatcaga agatggtagc 4380

atgtcgataa aaaatgaaga ggaagaacaa acactgacca tactcattag aacaggattg 4440

ctggtgatct caggactttt tcctgtatca ataccaatca cggcagcagc atggtacctg 4500

tgggaagtga agaaacaacg ggccggagta ttgtgggatg ttccttcacc cccacccatg 4560

ggaaaggctg aactggaaga tggagcctat agaattaagc aaaaagggat tcttggatat 4620

tcccagatcg gagccggagt ttacaaagaa ggaacattcc atacaatgtg gcatgtcaca 4680

cgtggcgctg ttctaatgca taaaggaaag aggattgaac catcatgggc ggacgtcaag 4740

aaagacctaa tatcatatgg aggaggctgg aagttagaag gagaatggaa ggaaggagaa 4800

gaagtccagg tattggcact ggagcctgga aaaaatccaa gagccgtcca aacgaaacct 4860

ggtcttttca aaaccaacgc cggaacaata ggtgctgtat ctctggactt ttctcctgga 4920

acgtcaggat ctccaattat cgacaaaaaa ggaaaagttg tgggtcttta tggtaatggt 4980

gttgttacaa ggagtggagc atatgtgagt gctatagccc agactgaaaa aagcattgaa 5040

gacaacccag agatcgaaga tgacattttc cgaaagagaa gactgaccat catggacctc 5100

cacccaggag cgggaaagac gaagagatac cttccggcca tagtcagaga agctataaaa 5160

cggggtttga gaacattaat cttggccccc actagagttg tggcagctga aatggaggaa 5220

gcccttagag gacttccaat aagataccag accccagcca tcagagctgt gcacaccggg 5280

cgggagattg tggacctaat gtgtcatgcc acatttacca tgaggctgct atcaccagtt 5340

agagtgccaa actacaacct gattatcatg gacgaagccc atttcacaga cccagcaagt 5400

atagcagcta gaggatacat ctcaactcga gtggagatgg gtgaggcagc tgggattttt 5460

atgacagcca ctcccccggg aagcagagac ccatttcctc agagcaatgc accaatcata 5520

gatgaagaaa gagaaatccc tgaacgctcg tggaattccg gacatgaatg ggtcacggat 5580

tttaaaggga agactgtttg gttcgttcca agtataaaag caggaaatga tatagcagct 5640

tgcctgagga aaaatggaaa gaaagtgata caactcagta ggaagacctt tgattctgag 5700

tatgtcaaga ctagaaccaa tgattgggac ttcgtggtta caactgacat ttcagaaatg 5760

ggtgccaatt tcaaggctga gagggttata gaccccagac gctgcatgaa accagtcata 5820

ctaacagatg gtgaagagcg ggtgattctg gcaggaccta tgccagtgac ccactctagt 5880

gcagcacaaa gaagagggag aataggaaga aatccaaaaa atgagaatga ccagtacata 5940

tacatggggg aacctctgga aaatgatgaa gactgtgcac actggaaaga agctaaaatg 6000

ctcctagata acatcaacac gccagaagga atcattccta gcatgttcga accagagcgt 6060

gaaaaggtgg atgccattga tggcgaatac cgcttgagag gagaagcaag gaaaaccttt 6120

gtagacttaa tgagaagagg agacctacca gtctggttgg cctacagagt ggcagctgaa 6180

ggcatcaact acgcagacag aaggtggtgt tttgatggag tcaagaacaa ccaaatccta 6240

gaagaaaacg tggaagttga aatctggaca aaagaagggg aaaggaagaa attgaaaccc 6300

agatggttgg atgctaggat ctattctgac ccactggcgc taaaagaatt taaggaattt 6360

gcagccggaa gaaagtctct gaccctgaac ctaatcacag aaatgggtag gctcccaacc 6420

ttcatgactc agaaggcaag agacgcactg gacaacttag cagtgctgca cacggctgag 6480

gcaggtggaa gggcgtacaa ccatgctctc agtgaactgc cggagaccct ggagacattg 6540

cttttactga cacttctggc tacagtcacg ggagggatct tttattcttt gatgagcgca 6600

aggggcatag ggaagatgac cctgggaatg tgctgcataa tcacggctag catcctccta 6660

tggtacgcac aaatacagcc acactggata gcagcttcaa taatactgga gttttttctc 6720

atagttttgc ttattccaga acctgaaaaa cagagaacac cccaagacaa ccaactgacc 6780

tacgttgtca tagccatcct cacagtggtg gccgcaacca tggcaaacga gatgggtttc 6840

ctagaaaaaa cgaagaaaga tctcggattg ggaagcattg caacccagca acccgagagc 6900

aacatcctgg acatagatct acgtcctgca tcagcatgga cgctgtatgc cgtggccaca 6960

acatttgtta caccaatgtt gagacatagc attgaaaatt cctcagtgaa tgtgtcccta 7020

acagctatag ccaaccaagc cacagtgtta atgggtctcg ggaaaggatg gccattgtca 7080

aagatggaca tcggagttcc ccttctcgcc attggatgct actcacaagt caaccccata 7140

actctcacag cagctctttt cttattggta gcacattatg ccatcatagg gccaggactc 7200

caagcaaaag caaccagaga agctcagaaa agagcagcgg cgggcatcat gaaaaaccca 7260

actgtcgatg gaataacagt gattgaccta gatccaatac cttatgatcc aaagtttgaa 7320

aagcagttgg gacaagtaat gctcctagtc ctctgcgtga ctcaagtatt gatgatgagg 7380

actacatggg ctctgtgtga ggctttaacc ttagctaccg ggcccatctc cacattgtgg 7440

gaaggaaatc cagggaggtt ttggaacact accattgcgg tgtcaatggc taacattttt 7500

agagggagtt acttggccgg agctggactt ctcttttcta ttatgaagaa cacaaccaac 7560

acaagaaggg gaactggcaa cataggagag acgcttggag agaaatggaa aagccgattg 7620

aacgcattgg gaaaaagtga attccagatc tacaagaaaa gtggaatcca ggaagtggat 7680

agaaccttag caaaagaagg cattaaaaga ggagaaacgg accatcacgc tgtgtcgcga 7740

ggctcagcaa aactgagatg gttcgttgag agaaacatgg tcacaccaga agggaaagta 7800

gtggacctcg gttgtggcag aggaggctgg tcatactatt gtggaggact aaagaatgta 7860

agagaagtca aaggcctaac aaaaggagga ccaggacacg aagaacccat ccccatgtca 7920

acatatgggt ggaatctagt gcgtcttcaa agtggagttg acgttttctt catcccgcca 7980

gaaaagtgtg acacattatt gtgtgacata ggggagtcat caccaaatcc cacagtggaa 8040

gcaggacgaa cactcagagt ccttaactta gtagaaaatt ggttgaacaa caacactcaa 8100

ttttgcataa aggttctcaa cccatatatg ccctcagtca tagaaaaaat ggaagcacta 8160

caaaggaaat atggaggagc cttagtgagg aatccactct cacgaaactc cacacatgag 8220

atgtactggg tatccaatgc ttccgggaac atagtgtcat cagtgaacat gatttcaagg 8280

atgttgatca acagatttac aatgagatac aagaaagcca cttacgagcc ggatgttgac 8340

ctcggaagcg gaacccgtaa catcgggatt gaaagtgaga taccaaacct agatataatt 8400

gggaaaagaa tagaaaaaat aaagcaagag catgaaacat catggcacta tgaccaagac 8460

cacccataca aaacgtgggc ataccatggt agctatgaaa caaaacagac tggatcagca 8520

tcatccatgg tcaacggagt ggtcaggctg ctgacaaaac cttgggacgt cgtccccatg 8580

gtgacacaga tggcaatgac agacacgact ccatttggac aacagcgcgt ttttaaagag 8640

aaagtggaca cgagaaccca agaaccgaaa gaaggcacga agaaactaat gaaaataaca 8700

gcagagtggc tttggaaaga attagggaag aaaaagacac ccaggatgtg caccagagaa 8760

gaattcacaa gaaaggtgag aagcaatgca gccttggggg ccgtattcac tgatgagaac 8820

aagtggaagt cggcacgtga ggctgttgaa gatagtaggt tttgggagct ggttgacaag 8880

gaaaggaatc tccatcttga aggaaagtgt gaaacatgtg tgtacaacat gatgggaaaa 8940

agagagaaga agctagggga attcggcaag gcaaaaggca gcagagccat atggtacatg 9000

tggcttggag cacgcttctt agagtttgaa gccctaggat tcttaaatga agatcactgg 9060

ttctccagag agaactccct gagtggagtg gaaggagaag ggctgcacaa gctaggttac 9120

attctaagag acgtgagcaa gaaagaggga ggagcaatgt atgccgatga caccgcagga 9180

tgggatacaa gaatcacact agaagaccta aaaaatgaag aaatggtaac aaaccacatg 9240

gaaggagaac acaagaaact agccgaggcc attttcaaac taacgtacca aaacaaggtg 9300

gtgcgtgtgc aaagaccaac accaagaggc acagtaatgg acatcatatc gagaagagac 9360

caaagaggta gtggacaagt tggcacctat ggactcaata ctttcaccaa tatggaagcc 9420

caactaatca gacagatgga gggagaagga gtctttaaaa gcattcagca cctaacaatc 9480

acagaagaaa tcgctgtgca aaactggtta gcaagagtgg ggcgcgaaag gttatcaaga 9540

atggccatca gtggagatga ttgtgttgtg aaacctttag atgacaggtt cgcaagcgct 9600

ttaacagctc taaatgacat gggaaagatt aggaaagaca tacaacaatg ggaaccttca 9660

agaggatgga atgattggac acaagtgccc ttctgttcac accatttcca tgagttaatc 9720

atgaaagacg gtcgcgtact cgttgttcca tgtagaaacc aagatgaact gattggcaga 9780

gcccgaatct cccaaggagc agggtggtct ttgcgggaga cggcctgttt ggggaagtct 9840

tacgcccaaa tgtggagctt gatgtacttc cacagacgcg acctcaggct ggcggcaaat 9900

gctatttgct cggcagtacc atcacattgg gttccaacaa gtcgaacaac ctggtccata 9960

catgctaaac atgaatggat gacaacggaa gacatgctga cagtctggaa cagggtgtgg 10020

attcaagaaa acccatggat ggaagacaaa actccagtgg aatcatggga ggaaatccca 10080

tacttgggga aaagagaaga ccaatggtgc ggctcattga ttgggttaac aagcagggcc 10140

acctgggcaa agaacatcca agcagcaata aatcaagtta gatcccttat aggcaatgaa 10200

gaatacacag attacatgcc atccatgaaa agattcagaa gagaagagga agaagcagga 10260

gttctgtggt agaaagcaaa actaacatga aacaaggcta gaagtcaggt cggattaagc 10320

catagtacgg aaaaaactat gctacctgtg agccccgtcc aaggacgtta aaagaagtca 10380

ggccatcata aatgccatag cttgagtaaa ctatgcagcc tgtagctcca cctgagaagg 10440

tgtaaaaaat ccgggaggcc acaaaccatg gaagctgtac gcatggcgta gtggactagc 10500

ggttagagga gacccctccc ttacaaatcg cagcaacaat gggggcccaa ggcgagatga 10560

agctgtagtc tcgctggaag gactagaggt tagaggagac ccccccgaaa caaaaaacag 10620

catattgacg ctgggaaaga ccagagatcc tgctgtctcc tcagcatcat tccaggcaca 10680

gaacgccaga aaatggaatg gtgctgttga atcaacaggt tct 10723

<210> 24
<211> 20
<212> DNA
<213> artificial

<220>
<223> artificial

<400> 24
ccgactacgg aacccttaca 20

<210> 25
<211> 23
<212> DNA
<213> artificial

<220>
<223> artificial

<400> 25
ggtctcagcc acttctttct cta 23

<210> 26
<211> 22
<212> DNA
<213> artificial

<220>
<223> artificial

<400>
gccatgcaca cacagcactt ac 22

<210> 27
<211> 20
<212> DNA
<213> artificial

<220>
<223> artificial

<400> 27
cttcagttgt cccggctcta 20

<210>  28
<211>  19
<212>  DNA
<213>  artificial

<220>
<223>  artificial

<400>  28
aagcccacgc tggacatag                                                    19


<210>  29
<211>  20
<212>  DNA
<213>  artificial

<220>
<223>  artificial

<400>  29
aagtgccccg caaagatact                                                   20

<210>  30
<211>  21
<212>  DNA
<213>  artificial

<220>
<223>  artificial

<400>  30
gccggagaga tgtggtagac                                                   21


<210>  31
<211>  21
<212>  DNA
<213>  artificial

<220>
<223>  artificial

<400>  31
ctgggagtta tcgtggctgta                                                  21